(19)

**Europäisches Patentamt**
**European Patent Office**
**Office européen des brevets**

(11) **EP 4 574 164 A1**

(12) **EUROPEAN PATENT APPLICATION**

(43) Date of publication:
**25.06.2025 Bulletin 2025/26**

(21) Application number: **23219776.4**

(22) Date of filing: **22.12.2023**

(51) International Patent Classification (IPC):
**A61K 38/18** (2006.01)     **A61P 19/08** (2006.01)

(52) Cooperative Patent Classification (CPC):
**A61K 38/1875; A61P 19/08**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Universität Bern**
**3012 Bern (CH)**

(72) Inventors:
• **ALBERS, Christoph**
**3006 Bern (CH)**

• **BIGDON, Sebastian**
**3033 Bern (CH)**
• **GANTENBEIN, Benjamin**
**3005 Bern (CH)**
• **RUTSCH, Niklas**
**5000 Aarau (CH)**

(74) Representative: **HGF**
**HGF Limited**
**1 City Walk**
**Leeds LS11 9DX (GB)**

Remarks:
The complete document including Reference Table(s) and the Sequence Listing(s) can be downloaded from the EPO website

(54) **COMBINATION OF A BMP AND BMP2 L51P**

(57)     The present invention relates to a combination comprising an osteogenic bone morphogenic protein (BMP) and a BMP antagonist inhibitor. The combinations are particularly useful in methods of spinal fusion, wherein the combinations are locally applied to the site of spinal fusion.

EP 4 574 164 A1

## Description

The present invention relates to a combination comprising an osteogenic bone morphogenic protein (BMP) and a BMP antagonist inhibitor. The combinations are particularly useful in methods of spinal fusion, wherein the combinations are locally applied to the site of spinal fusion.

## BACKGROUND OF THE INVENTION

Spinal fusion is one of the most frequently performed surgical interventions in spine surgery [1,2], with over two million surgeries performed worldwide [3]. The goal is to join one or more functional segments of the spine, to eliminate motion between the segments and increase stability [1,2,4-6]. Back pain caused by disc degeneration or segmental instability can be treated with spinal fusion [7-9]. However, failure of spinal fusion can lead to pseudoarthrosis resulting in persistent instability and pain [10].

The strong osteoinductive feature of bone morphogenic protein 2 (BMP2) was first described by Urist in (1965) [11]. BMP2 is known to induce osteogenesis and is applied clinically to enhance spinal fusion. BMP2, however, needs to be used in high doses to be effective, which can result in significant adverse effects. Two BMPs, i.e., BMP2, BMP6 and BMP7, are frequently employed to improve osteoinductivity, promote spinal fusion, and decrease the risk of pseudoarthrosis [12-14]. For example, BMP6 was used for spinal fusion applications and considered to be more resistant to Noggin (a prominent BMP inhibitor) inhibition. BMPs are a complex family of cytokines with complex pathways and multiple BMP inhibitors have been described such as inhibitors of the differential screening-selected gene aberrative in neuroblastoma (DAN) family, the Chordin family (e.g., Chordin and Noggin), and Follistatin, and Twisted Gastrulation have been recently discussed in detail. Despite their use, failure of osseous fusion has been reported in 5-35.5% of patients, with up to 23.6% of patients requiring revision surgery [7,9,15]. It is hypothesised that one reason for osseous fusion failure is due to the presence of BMP antagonists in the disc niche [16].

Previous *in vitro* data revealed that intervertebral disc (IVD) cells exert inhibitory effects on the osteogenic differentiation of mesenchymal stem cells (MSCs) [17,18]. Similar effects of disc cells could be shown on primary osteoblasts from patients [19]. Additionally, clinical trials revealed decreased fusion rates after incomplete removal of IVD cells [16,20,21]. These results suggest that endogenous BMP inhibitors, like Gremlin, Noggin, Chordin and Follistatin, of these cells might be crucial for prevention of proper fusion.

Thus, there remains a need to improve the effectiveness of spinal fusion, to reduce postoperative complications and the need for revision surgery, and to lower the associated health costs.

L51P is a BMP2 analogue that acts by inhibition of BMP2 inhibitors. In particular, L51P has been shown to promote differentiation exclusively through inhibiting BMP2 antagonists in an *in-vivo* critical bone size defect model in femurs of C57Bl/6J mice [22,23]. It is hypothesised that L51P acts through competitive blocking of BMP inhibitors, thus occupying inhibitors such as Noggin, Chordin or Gremlin [24,26,27]. The inhibitory effects of IVD cells on osteoblast differentiation has also been successfully reversed by adding L51P [18,19].

As described herein, the inventors have surprisingly found that combinations comprising BMP2 and the BMP2-analogue L51P show an improved spinal fusion outcome. In particular, the inventors have shown that the combinations can advantageously increase the efficacy of BMP2, and lower the required doses for spinal fusion.

## BRIEF SUMMARY OF THE DISCLOSURE

In accordance with the present invention, there is provided in a first aspect a combination comprising an osteogenic bone morphogenic protein (BMP) and a BMP antagonist inhibitor for use in a method of spinal fusion in a subject, the method comprising locally applying the combination to the site of spinal fusion; wherein the BMP antagonist inhibitor is L51P.

In a second aspect, the present invention provides a combination comprising an osteogenic bone morphogenic protein (BMP) and a BMP antagonist inhibitor for use in treating conditions of the spine in a subject, wherein the combination is used in a treatment method of spinal fusion; wherein the BMP antagonist inhibitor is L51P.

In a third aspect, the present invention provides a combination comprising an osteogenic bone morphogenic protein (BMP) and a BMP antagonist inhibitor for use in treating spinal stenosis, spondylolisthesis, spondylosis, spinal fractures, scoliosis, and kyphosis in a subject, wherein the combination is used in a treatment method of spinal fusion; wherein the BMP antagonist inhibitor is L51P.

In a forth aspect, there is provided L51P for use in a method of spinal fusion in a subject, the method comprising locally applying the L51P to the site of spinal fusion concurrently (e.g. simultaneously or sequentially) with an osteogenic bone morphogenic protein (e.g. BMP-2 ).

In a fifth aspect, there is provided an osteogenic bone morphogenic protein (e.g. BMP-2) for use in a method of spinal fusion in a subject, the method comprising locally applying the osteogenic bone morphogenic protein to the site of spinal fusion concurrently (e.g. simultaneously or sequentially) with L51P.

In a sixth aspect, there is provided a combination product comprising an intervertebral spacer or carrier dimensioned for a human intervertebral disc-space, an osteogenic bone morphogenic protein (e.g. BMP-2), and a BMP antagonist inhibitor (e.g. L51P).

In a seventh aspect, a kit comprising: (i) an intervertebral spacer or carrier dimensioned for a human intervertebral disc-space; (ii) an osteogenic bone morphogenic protein (e.g. BMP-2); and (iii) a BMP antagonist inhibitor (e.g. L51P).

In an eight aspect the present invention provides a kit comprising:

(i) an osteogenic bone morphogenic protein (e.g. BMP-2);

(ii) a BMP antagonist inhibitor (e.g. L51P); and

(iii) instructions for use.

## BRIEF DESCRIPTION OF THE DRAWINGS

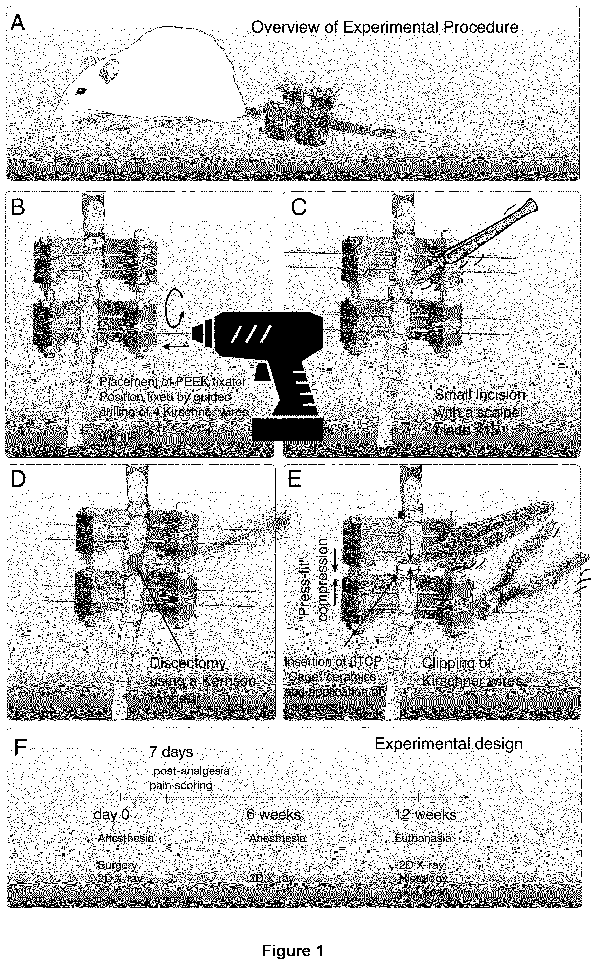

Figure 1 - Overview of Experimental Procedure. Sketch illustrating the standard operational procedure (SOP) for spinal surgery in elderly rats for spinal fusion models. (a) Overview of external ring fixator (Ex-Fix) mounted on adult Wistar rat; (b) Mounting of Ex-Fix by drilling four Kirschner wires through two adjacent vertebrae; (c) Midline incision using a small scalpel blade, e.g. #15; (d) Discectomy with a Kerrison rongeur; (e) Insertion of βTCP carrier (± coated with BMPs). This procedure was followed by press-fit compression using the Ex-Fix, finalised by a two-step-wound closure and clipping of the K-wires; (f) Experimental design of the 12-weeks post-operative follow-up and read-out parameters. Parts of the figure has been republished with permission from [28] ©CC-YY, MDPI, Basel, Switzerland.

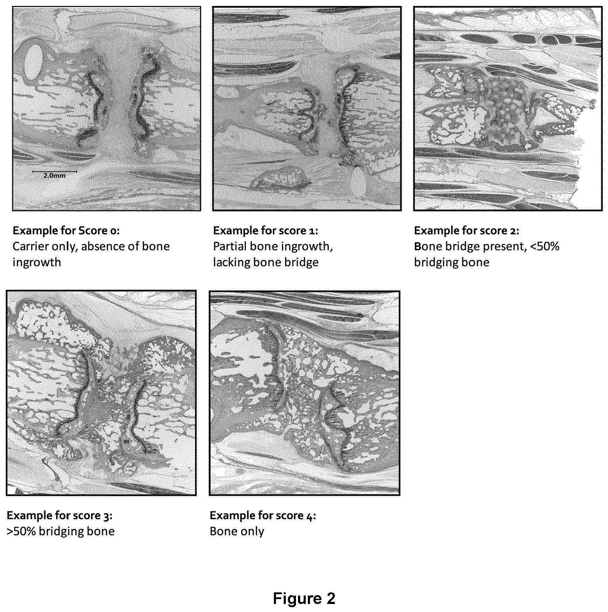

Figure 2 - Representative images of PMMA-embedded thick sections and applied semi-quantitative histology scoring system based on Emery *et al.* (1994) [37]. Staining: toluidine blue/fuchsin red.

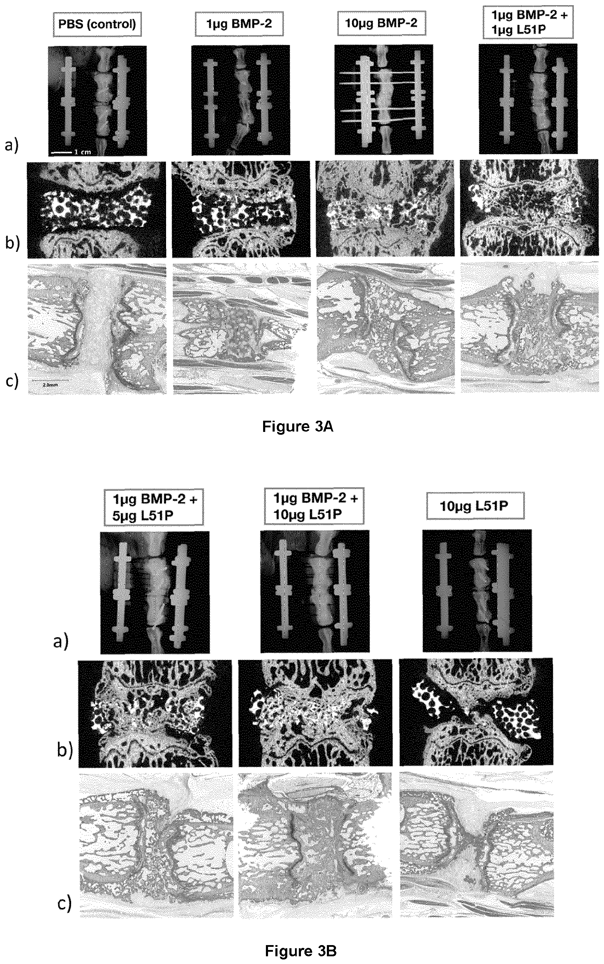

Figures 3A and 3B - Overview of the results of the proximal tail region of coccygeal spinal fusion model of elderly Wistar rats twelve weeks post-operatively, showing: a) one representative image per 2D X-ray, apical position (Faxitron); b) Mid-sagittal plane section of μCT images, in white colour remaining βTCP carrier, in grey colour bone; c) histology of PMMA-embedded thick sections (-200 μm).

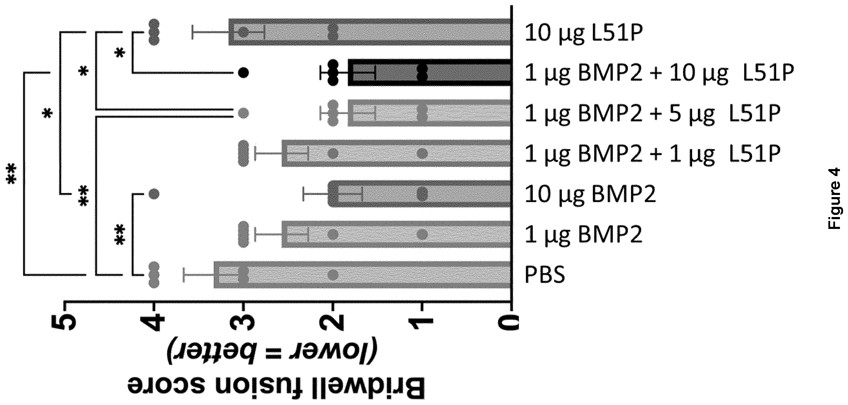

Figure 4 - Semi-quantitative analysis of spinal fusion model of elderly Wistar rats by Bridwell scores [32]. Two independent orthopaedic surgeons quantified the X-ray images (0 is best and 4 is worst). Shown are means ± SEM. Stars indicate level of statistical significance; *: $P < 0.05$, **: $P < 0.025$, ***: $P < 0.01$.

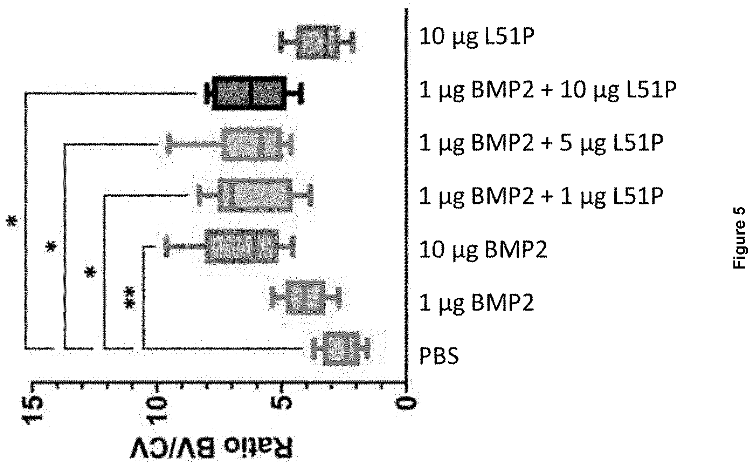

Figure 5 - Whisker plots of bone volume relative to ceramics volume (βTCP) (BV/CV) of μCT data. Shown are min to max and medians. Stars indicate level of statistical significance; *: $P < 0.05$, **: $P < 0.025$, ***: $P < 0.01$.

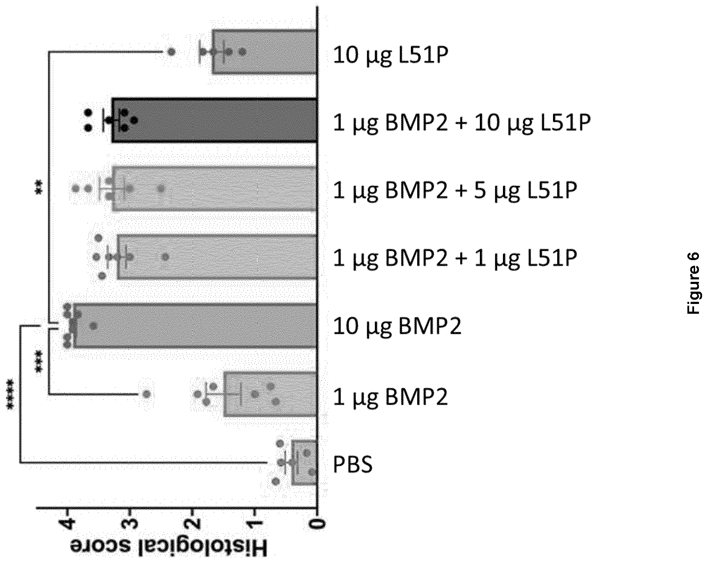

Figure 6 - Bar plots of histology scoring of PMMA-thick sections of the coccygeal spinal fusion model of elderly Wistar rat according to Emery *et al.* (1994) [37]. Shown are the means ± SEM of the histological scores of three independent scorers. Stars indicate level of statistical significance; *: $P < 0.05$, **: $P < 0.025$, ***: $P < 0.01$.

## DETAILED DESCRIPTION

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

For the avoidance of doubt, the information disclosed earlier in this specification under the heading "Background" is relevant to the invention and is to be read as part of the disclosure of the invention.

The reader's attention is directed to all papers and documents which are filed concurrently with or previous to this specification in connection with this application and which are open to public inspection with this specification, and the contents of all such papers and documents are incorporated herein by reference.

In a first aspect of the invention there is provided, a combination comprising an osteogenic bone morphogenic protein (BMP) and a BMP antagonist inhibitor for use in a method of spinal fusion in a subject, the method comprising locally applying the combination to the site of spinal fusion; wherein the BMP antagonist inhibitor is L51P.

Spinal fusion, also referred to as spondylodesis or spondylosyndesis, is a surgery performed by orthopaedic surgeons or neurosurgeons that joins two or more vertebrae. This procedure can be performed at any level in the spine (cervical, thoracic, lumbar, or sacral) and prevents movement between the fused vertebrae. There are many types of spinal fusion and each technique involves using bone grafting-either from the patient (autograft), donor (allograft), or artificial bone substitutes-to help the bones heal together. Additional hardware (screws, plates, or cages) is often used to hold the bones in place while the graft fuses the two vertebrae together. The placement of hardware can be guided by fluoroscopy, navigation systems, or robotics. Spinal fusion can be used to treat a variety of conditions affecting any level of the spine-lumbar, cervical and thoracic. In general, spinal fusion is performed to decompress and stabilize the spine. The most common cause of pressure on the spinal cord/nerves is degenerative disc disease. Other common causes include disc herniation, spinal stenosis, trauma, and spinal tumors. Spinal stenosis results from bony growths (osteophytes) or thickened ligaments that cause narrowing of the spinal canal over time. This causes leg pain with increased activity, a condition called neurogenic claudication. Pressure on the nerves as they exit the spinal cord (radiculopathy) causes pain in the area where the nerves originated (leg for lumbar pathology, arm for cervical pathology). In severe cases, this pressure can cause neurologic deficits, like numbness, tingling, bowel/bladder dysfunction, and paralysis. Lumbar and cervical spinal fusions are more commonly performed than thoracic fusions. Degeneration happens more frequently at these levels due to increased motion and stress. The thoracic spine is more immobile, so most fusions are performed due to trauma or deformities like scoliosis, kyphosis, and lordosis.

Conditions where spinal fusion may be considered include the following: degenerative disc disease, spinal disc herniation, discogenic pain, spinal tumor, vertebral fracture, scoliosis, kyphosis (e. g., Scheuermann's disease), lordosis, spondylolisthesis, spondylosis, posterior rami syndrome, other degenerative spinal conditions, or any condition that causes instability of the spine.

In certain aspects of the present invention the BMPs may be, for instance, endogenous BMPs found naturally in the body, or may be natural BMPs added to the treatment site. In other aspects of the present invention, for instance, the BMPs may be or may include recombinant BMPs.

Suitable BMPs include BMP-2, BMP-5, BMP-4, BMP-6 and BMP-7.

A typical group of BMPs includes BMP-5, BMP-6, BMP7, BMP8/OP-2 and BMP-8B. Another typical group of BMPs include BMP-2 and BMP-4. Another typical group of BMPs also include BMP3 and BMP3B/GDF-10. Also, a typical group of BMPs include GDF-5/CDMP-1/BMP-14, GDF-6/CDMP-2/BMP13, GDF-7/CDMP-3/BMP-12. Typically the BMP may be GDF-9. Also the BMP may be GDF3 in other embodiments of the invention. Aptly the BMPs of the invention may include BMP-2, BMP-4, BMP-6 and BMP-7.

The BMPs may include, BMP-2 in certain embodiments of the present invention. Or may include BMP-4 in certain embodiments of the present invention. Alternatively in other embodiments of the present invention the BMP may be BMP-7. Likewise in other embodiments the BMP may be BMP-6.

In one embodiment, the osteogenic BMP is selected from BMP-2, BMP-4, BMP-5, BMP-7, and BMP-9.

In a preferred embodiment the osteogenic BMP is selected from BMP-2 and BMP-7.

In another embodiment the osteogenic BMP is BMP-2.

In preferred embodiment the wild type BMP comprises nucleic acid sequences:

hBMP-2 comprises a nucleic acid sequence according to SEQ ID No. 1;

hBMP-4 comprises a nucleic acid sequence according to SEQ ID No. 2;

hBMP-5 comprises a nucleic acid sequence according to SEQ ID No. 3;

hBMP-6 comprises a nucleic acid sequence according to SEQ ID No. 4;

hBMP-7 comprises a nucleic acid sequence according to SEQ ID No. 5;

hBMP-8 comprises a nucleic acid sequence according to SEQ ID No. 6;

hGDF-5 comprises a nucleic acid sequence according to SEQ ID No. 7;

mGDF-6 comprises a nucleic acid sequence according to SEQ ID No. 8;

mGDF-7 comprises a nucleic acid sequence according to SEQ ID No. 9;

hBMP-10 comprises a nucleic acid sequence according to SEQ ID No. 10; and

hGDF-2 comprises a nucleic acid sequence according to SEQ ID No. 11.

The term "nucleic acid" as used herein also comprises any fragments of the nucleic acid as described herein, whereby preferably any such fragment comprises a length from about 19 to 30, more preferably 19 to 25 and most preferably 21 and 22 consecutive nucleotides. Even more preferably this kind of fragment of a nucleic acid is present as a double-stranded structure.

In preferred embodiment the wild type BMP comprises amino acid sequences:

hBMP-2 comprises an amino acid sequence according to SEQ ID No. 13;

hBMP-4 comprises an amino acid sequence according to SEQ ID No. 14;

hBMP-5 comprises an amino acid sequence according to SEQ ID No. 15;

hBMP-6 comprises an amino acid sequence according to SEQ ID No. 16;

hBMP-7 comprises an amino acid sequence according to SEQ ID No. 17;

hBMP-8 comprises an amino acid sequence according to SEQ ID No. 18;

hGDF-5 comprises an amino acid sequence according to SEQ ID No. 19;

mGDF-6 comprises an amino acid sequence according to SEQ ID No. 20;

mGDF-7 comprises an amino acid sequence according to SEQ ID No. 21;

hBMP-10 comprises an amino acid sequence according to SEQ ID No. 22; and

hGDF-2 comprises an amino acid sequence according to SEQ ID No. 23.

In another embodiment the osteogenic BMP comprises an amino acid sequence or nucleic acid sequence according to any of SEQ ID Nos. 1 to 11 and 13 to 23 or a variant thereof. In a preferred embodiment the osteogenic BMP comprises greater than 90%, 85%, 75%, 70%, 65% sequence identity to an amino acid sequence or nucleic acid sequence according to any of SEQ ID Nos. 1 to 11 and 13 to 23 or a variant thereof. In a preferred embodiment the osteogenic BMP comprises having at least 80%, 85%, 90%, 95%, 97%, 99%, or 100% sequence identity to SEQ ID NO: 1 to 11 and 13 to 23 or variant thereof.

The term "bone morphogenetic protein" also comprises orthologues thereof. As used herein, an orthologue is a protein from another organism that fulfils the same genetic and physiological function as the reference protein. Also, in a more preferred embodiment, the term "bone morphogenetic protein" comprises any truncated protein and mutant, respectively. Preferably, such truncated protein and mutant lacks at least one amino acid residue, preferably at either the N terminus or the C terminus.

A soluble, BMP polypeptide may include one, two, three, four, five or more alterations in the amino acid sequence relative to a naturally occurring BMP polypeptide. The alteration in the amino acid sequence may, for example, alter glycosylation of the polypeptide when produced in a mammalian, insect or other eukaryotic cell or alter proteolytic cleavage of the polypeptide relative to the naturally occurring BMP polypeptide.

The terms "peptide," "polypeptide," and "protein" are used interchangeably and refer to any chain of two or more natural or unnatural amino acid residues, regardless of post-translational modification (e.g., glycosylation or phosphorylation), constituting all or part of a naturally-occurring or non-naturally occurring polypeptide or peptide, as is described herein.

In one embodiment the osteogenic BMP is a recombinant BMP, preferably a recombinant human BMP.

In one embodiment the BMP antagonist inhibitor is recombinant human L51P.

In another embodiment the osteogenic BMP is recombinant human BMP-2 and the BMP antagonist inhibitor is recombinant human L51P.

L51P is a BMP2-analogue.

Functionally active fragments and analogues may be formed by the addition, insertion, modification, substitution or deletion of one or more of the amino acid residues from or to an amino acid sequence described herein.

The term "analogue" is also intended to embrace chimeric proteins, fusion proteins, ant idiotypic antibodies, precursor and other functional equivalents or mimics to the above.

L51P is deficient in type I receptor binding, whereas its overall structure and its binding to type II receptors and modulator proteins, including Noggin, Gremlin, and Chordin, are unchanged. These modifications make L51P a BMP receptor-inactive inhibitor of endogenous BMP antagonists.

BMP2 mutant L51P comprises a nucleic acid sequence according to SEQ ID No. 12 and/or comprises an amino acid sequence according to SEQ ID No. 24. In another embodiment the BMP2 mutant L51P comprises an amino acid sequence or nucleic acid sequence according to any of SEQ ID Nos. 12 or 24 or a variant thereof. In a preferred embodiment the BMP2 mutant L51P comprises greater than 90%, 85%, 75%, 70%, 65% sequence identity to an amino acid sequence or nucleic acid sequence according to any of SEQ ID Nos. 12 or 24 or a variant thereof. In a preferred embodiment the osteogenic BMP comprises having at least 80%, 85%, 90%, 95%, 97%, 99%, or 100% sequence identity to SEQ ID NO: 12 or 24 or variant thereof.

Identity with respect to a sequence is defined herein as the percentage of amino acid residues in the candidate sequence that are identical with the reference amino acid sequence after aligning the sequences and introducing gaps, if necessary, to achieve the maximum percent sequence identity, and not considering any conservative substitutions as part of the sequence identity.

Sequence identity can be determined by standard methods that are commonly used to compare the similarity in position of the amino acids of two polypeptides. Using a computer program such as BLAST or FASTA, two polypeptides are aligned for optimal matching of their respective amino acids (either along the full length of one or both sequences or along a predetermined portion of one or both sequences).

The skilled person will recognise that individual substitutions, deletions or additions to a protein which alters, adds or deletes a single amino acid or a small percentage of amino acids is a "derivative" where the alteration(s) results in the substitution of an amino acid with a functionally similar amino acid or the substitution/deletion/addition of residues which do not impact the function.

Conservative substitution tables providing functionally similar amino acids are well known in the art. In general, such conservative substitutions will fall within one of the aminoacid groupings specified below, though in some circumstances other substitutions may be possible without substantially affecting the properties of the protein. The following eight groups each contain amino acids that are typically conservative substitutions for one another:

1) Alanine (A), Glycine (G);

2) Aspartic acid (D), Glutamic acid (E);

3) Asparagine (N), Glutamine (Q);

4) Arginine (R), Lysine (K);

5) Isoleucine (I), Leucine (L), Methionine (M), Valine (V);

6) Phenylalanine (F), Tyrosine (Y), Tryptophan (W);

7) Serine (S), Threonine (T); and

8) Cysteine (C), Methionine (M)

BMP and L51P gene sequences, protein sequences, and methods for producing recombinant and naturally-derived BMPs and L51P are known in the art.

In one embodiment the molar ratio of L51P to osteogenic BMP is less than 10:1, or is less than or equal to about 5:1; preferably wherein the molar ratio of L51P to osteogenic BMP is less than 3:1 or is 1:1 or less than 1:1.

In one embodiment the osteogenic BMP is applied to the site of spinal fusion at a dose of less than 2 mg per spinal fusion, or a dose of from about 0.1 mg to about 0.5 mg, or 0.1 mg to about 1 mg, or 0.01 to 0.1mg, or 0.5mg to 1mg, or 1 mg to about 2mg per spinal fusion.

In one embodiment the osteogenic BMP and the BMP antagonist inhibitor are locally applied to the site of spinal fusion simultaneously or sequentially.

In another embodiment the osteogenic BMP and the BMP antagonist inhibitor are comprised in one or more pharmaceutical composition, for example wherein the pharmaceutical composition is selected from a liquid, gel, suspension, paste, putty, powder, granule, bead, an implant and a polyelectrolyte complex; optionally wherein

(i) the osteogenic BMP and the BMP antagonist inhibitor are in separate pharmaceutical compositions; or

(ii) the osteogenic BMP and the BMP antagonist inhibitor are in a single pharmaceutical composition.

Such pharmaceutical composition according to the present invention also comprises a pharmaceutically acceptable carrier. A pharmaceutically acceptable carrier is preferably either a liquid or a solid carrier. Suitable liquid carriers are, among others, water, aqueous solutions, more preferably buffers, or lipids or lipid compositions. Preferred solid carriers comprise, among others, sugar, cellulose and starch. It is to be acknowledged that those skilled in the art, preferably in the art of formulations, are aware of further pharmaceutically acceptable carriers and carrier material, respectively. In a preferred embodiment the pharmaceutical composition comprises a further pharmaceutically active agent.

By "pharmaceutically acceptable carrier" or "pharmaceutically acceptable excipient" it is meant at least one carrier or excipient, respectively, which is physiologically acceptable to the treated patient while retaining the therapeutic properties of the compound with which it is administered. One exemplary pharmaceutically acceptable carrier substance is physiological saline.

By "pharmaceutical composition" it is meant a composition containing a polypeptide or nucleic acid molecule as described herein formulated with at least one pharmaceutically acceptable excipient, diluent, or carrier. The pharmaceutical composition may be manufactured or sold with the approval of a governmental regulatory agency as part of a therapeutic regimen for the treatment or prevention of a disease or event in a patient. Pharmaceutical compositions can be formulated, for example, for subcutaneous administration, intravenous administration (e.g., as a sterile solution free of particulate emboli and in a solvent system suitable for intravenous use), for oral administration (e.g., a tablet, capsule, caplet, gelcap, or syrup), or any other formulation described herein, e.g., in unit dosage form.

In another embodiment the mode of administration includes parenteral administration. "Parenteral administration," "administered parenterally," and other grammatically equivalent phrases, as used herein, refer to modes of administration other than enteral and topical administration, usually by injection, and include, without limitation, subcutaneous, intradermal, intravenous, intranasal, intraocular, pulmonary, intramuscular, intra-arterial, intrathecal, intracapsular, intraorbital, intracardiac, intradermal, intrapulmonary, intraperitoneal, transtracheal, subcuticular, intraarticular, subcapsular, subarachnoid, intraspinal, epidural, intracerebral, intracranial, intracarotid, and intrasternal injection and infusion.

In another embodiment the pharmaceutical composition comprises a therapeutically effective amount of osteogenic BMP and the BMP antagonist inhibitor. By "therapeutically effective amount" it is meant an amount of a polypeptide or nucleic acid molecule described herein that is sufficient to substantially improve, treat, prevent, delay, suppress, or arrest at least one symptom associated with spinal fusion. A therapeutically effective amount of a composition described herein may depend on the severity of the spinal fusion being treated and the condition, weight, and general state of the patient and can be determined by an ordinarily-skilled artisan with consideration of such factors. A therapeutically effective amount of a composition described herein can be administered to a patient in a single dose or in multiple doses administered over a period of time.

In one embodiment of the pharmaceutical composition is administered to a patient in an amount that is therapeutically effective to induce bone healing. Examples of inducing bone healing includes the surgical fusion of two joints. For example, the method is useful to promote spinal fusion. Spinal fusion surgery can be used to eliminate bone-on-bone friction and/or to relive nerve compression that causes pain and other symptoms. Surgical bone fusion can also be used on fingers, ankles, and feet, for instance.

By "treating," "treat," or "treatment" is meant the medical management of a patient with the intent to cure, ameliorate, stabilize, reduce the likelihood of, or prevent at least one symptoms associated with spinal fusion surgery.

Evaluation of Treatment Efficacy

Exemplary metrics useful for evaluating the efficacy of treatment using the pharmaceutical composition of the present invention include (1) bone healing, (2) bone mineralization, (3) bone mineral density, and (4) plasma PPi and PLP concentrations. The methods may further include the use of one or more of the described metrics (e.g., bone healing, mineralization, bone mineral density, or plasma PPi and PLP concentrations), singly or in combination, to assess treatment efficacy, in which improvements as described herein demonstrate that the pharmaceutical composition is an effective treatment. Additionally, the method may further include changing the dosage and/or frequency of the pharmaceutical composition administration in order to determine the effective amount of BMP and L51P to administer to a patient in need thereof.

Bone healing and mineralization

In some instances, administration of the pharmaceutical composition according to the present invention results in an increase in bone healing in the patient following the treatment. An increase in bone healing results in new bone, and includes the increased mineralization to result in the union of two or more bones (e.g., fusion of two normally separate bones, or union of two portions of a bone that have been separated by a fracture), or increased bone healing near instrumentation. Methods for identifying an increase in bone healing and mineralization are routine and include non-invasive techniques such as radiography and computed tomography (CT). Typically, images of the relevant area of the patient are taken before and at one or more time points following the treatment method, and the images are compared. Increased bone healing and/or mineralization can be identified as increased opacity. Pretreatment images can be taken at any time before the treatment method begins, and can be timed to be 1, 2, 3, 4, 5, or 6 day(s), week(s), or month(s) before treatment is initiated. Posttreatment images can be taken 1, 2, 3, 4, 5, 6, 7, 8, 9, 10, 11, or 12 day(s), week(s), month(s), or year(s) after treatment is initiated. The increase in bone healing and/or mineralization in the patient may become detectable at least 1, 2, 3, 4, 5, 6, 7, 8, 9, 10, 11, or 12 week(s) or month(s) following a treatment period. The increase in bone healing and/or mineralization in the patient may in some cases be sustained for at least 1, 2, 3, 4, 5, 6, 7, 8, 9, 10, 11, or 12 week(s), month(s), or year(s) following a treatment period. A reference bone can be one or more specific bones.

In another embodiment the pharmaceutical composition comprises the osteogenic BMP is present at a concentration from about 10% (w/w) to about 40% (w/w), 1% (w/w) to about 10% (w/w), or 10% (w/w) to 30% (w/w).

In another embodiment the pharmaceutical composition is a controlled release pharmaceutical composition; optionally wherein the pharmaceutical composition releases the osteogenic BMP and the BMP antagonist inhibitor over a period of at least 1-3 days, 1-5 days, 1-6 days, 1-7 days, 1-2 weeks, 1-3 weeks, 1-4 weeks, 2-5 weeks, 2-6 weeks. Controlled release can be measured as described according to the method found in the paper: Wernike et al., VEGF incorporated into calcium phosphate ceramics promotes vascularisation and bone formation in vivo. 2010, Feb, Eur Cell Mater 19: 30-40.

In another embodiment the osteogenic BMP and the BMP antagonist inhibitor are applied to the intervertebral disc space between the vertebrae to be fused.

In one embodiment the method of spinal fusion further comprises inserting a spacer or carrier into the intervertebral disc space between the vertebrae to be fused.

In another embodiment the spacer or carrier is selected from a solid implant, a cage, a porous matrix, a sponge, a scaffold, a bone allograft and a bone autograft.

In a preferred embodiment the spacer or carrier comprises an osteoconductive material.

In another embodiment the osteoconductive material is selected from a calcium phosphate ceramic (e.g. hydroxyapaptite, tricalcium phosphate, or biphasic calcium phosphate), a porous calcium carbonate (e.g. a coral-derived ceramic), a bioactive glass, a collagen sponge, an osteoconductive metal (e.g. porous titanium), an osteoconductive polymer (e.g. poly-hydroxyethylmethacrylate), and an osteoconductive composite (e.g. a poly-epsilon caprolactone tricalcium phosphate composite or a composite comprising hydroxyapaptite granules and a hydrogel comprising a tricalcium phosphate microspheres).

In a preferred embodiment the spacer or carrier comprises β-tricalcium phosphate, for example wherein the β-tricalcium phosphate has a porosity of 70-90%, from 60% to 90%, from 80 to 90% or from 90 to 95%. Bone porosity n can be defined (as described in EP2077759A2) as the ratio of a porous volume Wpor to an entire considered bone volume W by equation:

$$n = Wpor / W.$$

The values of porosity n for each cell of an observed volume are calculated by the modified WylHe relationship from oil geophysics. The calculations are done using calculated values of average longitudinal wave velocity value in a cell Vp, value longitudinal wave velocity in a matrix (skeleton) mineral part of an observed bone medium Vt, and the value longitudinal velocity in a filling -VfM, such way the material properties are estimated for example the porosity, elastic wave velocity and elastic wave velocity in matrix of an inspected bone.

In another embodiment wherein the spacer or carrier further comprises the osteogenic BMP and the BMP antagonist inhibitor (e.g. wherein the spacer or carrier is coated and/or infused with the osteogenic BMP and the BMP antagonist inhibitor).

In another embodiment the method of spinal fusion is selected from posterolateral interbody fusion, transforaminal interbody fusion, anterior interbody fusion and lateral lumbar interbody fusion; preferably wherein the method of spinal fusion is anterior interbody fusion.

In further embodiment the method of spinal fusion fuses two or more vertebrae selected from cervical vertebrae, thoracic vertebrae, lumber vertebrae and sacrum; preferably, wherein the method of spinal fusion fuses two or more lumbar vertebrae or fuses the L5 and S1 vertebrae.

In another embodiment wherein the method of spinal fusion further comprises laminectomy and/or discectomy.

In another embodiment the method of spinal fusion further comprises temporary immobilisation of the vertebrae to be fused; optionally wherein the vertebrae to be fused are immobilised by one or more screws, rods, wires or plates; preferably wherein the vertebrae to be fused are immobilised after application of the combination to the site of spinal fusion.

It is to be readily appreciated that the spinal fusion spacer or carrier of the present invention may be suitable for accomplishing fusion in the thoracic spinal surgery, cervical or lumbar spine. It should be noted that spacer or carriers designed for the cervical and lumbar regions may be composed in a similar manner to the thoracic spacer or carrier, but having a lordosis-promoting shape, rather than the kyphosis promoting shape. Specifically, the anterior side may be dimensioned to have a height greater than the posterior side. Those spacer or carrier designed to be inserted into the cervical region may be very similar to those to be inserted into the thoracic region, albeit smaller, while those designed for the lumbar region may be larger. Moreover, it is to be readily appreciated that the spacer or carriers described herein may be employed with any number of suitable constructions, including but not limited to metal, ceramic, plastic or composite.

In one embodiment the composition prevents or inhibits one or more of pseudoarthrosis, soft-tissue swelling, radiculitis, pain at the site of spinal fusion or ectopic bone formation, osteolysis, potential carcinogenic effects, and dysphagia at the cervical level.

In one embodiment the subject is a human or a non-human mammal (e.g. a horse or a companion animal (e.g. a cat or dog)), preferably wherein the subject is a human. In another embodiment the terms "patient" or "subject" refer to a mammal, including, but not limited to, a human or a non-human mammal, such as a bovine, equine, canine, ovine, or feline.

In another embodiment the subject is a smoker and/or has a co-morbidity selected from a metabolic disease (e.g. diabetes, smoking, obesity, medication) or osteoporosis or malnutrition. In another embodiment the subject has undergone chemotherapy, NSAIDS treatment, steroid treatment and other cancer treatments.

In another aspect, the present invention provides a combination comprising an osteogenic bone morphogenic protein (BMP) and a BMP antagonist inhibitor for use in treating conditions of the spine in a subject, wherein the combination is used in a treatment method of spinal fusion; wherein the BMP antagonist inhibitor is L51P.

In a preferred embodiment, the treatment of conditions of the spine, is to relieve pain and pressure from mechanical pain of the vertebrae or on the spinal cord. Conditions of the spine include spinal stenosis, spondylolisthesis, spondylosis, spinal fractures, scoliosis, and kyphosis. In another embodiment conditions of the spine include: degenerative disc disease, spinal disc herniation, discogenic pain, spinal tumor, vertebral fracture, scoliosis, kyphosis (e.g., Scheuermann's disease), lordosis, spondylolisthesis, spondylosis, posterior rami syndrome, other degenerative spinal conditions, and any condition that causes instability of the spine.

In another aspect, the present invention provides a combination comprising an osteogenic bone morphogenic protein (BMP) and a BMP antagonist inhibitor for use in treating spinal stenosis, spondylolisthesis, spondylosis, spinal fractures, scoliosis, and kyphosis in a subject, wherein the combination is used in a treatment method of spinal fusion; wherein the BMP antagonist inhibitor is L51P.

In another aspect, the present invention provides a L51P for use in a method of spinal fusion in a subject, the method comprising locally applying the L51P to the site of spinal fusion concurrently (e.g. simultaneously or sequentially) with an osteogenic bone morphogenic protein (e.g. BMP-2 ).

In another aspect, the present invention provides an osteogenic bone morphogenic protein (e.g. BMP-2) for use in a method of spinal fusion in a subject, the method comprising locally applying the osteogenic bone morphogenic protein to the site of spinal fusion concurrently (e.g. simultaneously or sequentially) with L51P.

In another aspect, the present invention provides a combination product comprising an intervertebral spacer or carrier dimensioned for a human intervertebral disc-space, an osteogenic bone morphogenic protein (e.g. BMP-2), and a BMP antagonist inhibitor (e.g. L51P).

In another aspect the present invention provides a kit comprising:

(i) an intervertebral spacer or carrier dimensioned for a human intervertebral disc-space;

(ii) an osteogenic bone morphogenic protein (e.g. BMP-2); and

(iii) a BMP antagonist inhibitor (e.g. L51P).

In another aspect the present invention provides a kit comprising:

(i) an osteogenic bone morphogenic protein (e.g. BMP-2);

(ii) a BMP antagonist inhibitor (e.g. L51P); and

(iii) instructions for use.

In other aspects kits are provided based on methods for the proteins of the present invention or their derivatives. The invention contemplates combinations of any of the foregoing aspects and embodiments of the invention. The present invention is further illustrated by the figures and examples from which further features, embodiments and advantages may be taken.

Sequence Listing

| Seq. ID NO: | Nucleic Acid |
|---|---|
| 1 (hBM P-2) | caagccaaacacaaacagcggaaacgccttaagtccagctgtaagagacacccttt gtacgtggacttcagtgacgtggggtggaatgactggattgtggctcccccggg gtatcacgcc ttttactgccacggagaatgccctttcctctggctgatcatctgaactccacta atcat gccattgttcagacgttggtcaactctgttaactctaagattcctaaggcatgct gtgtc ccgacagaactcagtgctatctcgatgctgtaccttgacgagaatgaaaaggttg tatta aagaactatcaggacatggttgtggagggttgtgggtgtcgctag |

(continued)

| Seq. ID NO: | Nucleic Acid |
|---|---|
| 2 (hBM P-4) | caagccaaacacaaacagcggaaacgccttaagtccagctgtaagagacaccctttgtacgtggacttcagtgacgtggggtggaatgactggattgtggctcccccggggtatcacgcc<br><br>ttttactgccacggagaatgccctttttcctctggctgatcatctgaactccactaatcat<br>gccattgttcagacgttggtcaactctgttaactctaagattcctaaggcatgctgtgtc<br>ccgacagaactcagtgctatctcgatgctgtaccttgacgagaatgaaaaggttgtatta<br>aagaactatcaggacatggttgtggagggttgtgggtgtcgctag<br><br>agccctaagcatcactcacagcgggccaggaagaagaataagaactgccggcgccactcg<br>ctctatgtggacttcagcgatgtgggctggaatgactggattgtggccccaccaggctac<br>caggccttctactgccatggggactgccccttttccactggctgaccacctcaactcaacc<br>aaccatgccattgtgcagaccctggtcaattctgtcaattccagtatccccaaagcctgt<br>tgtgtgcccactgaactgagtgccatctccatgctgtacctggatgagtatgataaggtg<br>gtactgaaaaattatcaggagatggtagtagagggatgtgggtgccgctga<br><br>aatcaaaaccgcaataaatccagctctcatcaggactcctccagaatgtccagtgttgga<br>gattataacacaagtgagcaaaaacaagcctgtaagaagcacgaactctatgtgagcttc<br>cgggatctgggatggcaggactggattatagcaccagaaggatacgctgcattttattgt<br>gatggagaatgttcttttccacttaacgcccatatgaatgccaccaaccacgctatagtt<br>cagactctggttcatctgatgtttcctgaccacgtaccaaagccttgttgtgctccaacc |
| 3 (hBM P-5) | aatcaaaaccgcaataaatccagctctcatcaggactcctccagaatgtccagtgttgga<br>gattataacacaagtgagcaaaaacaagcctgtaagaagcacgaactctatgtgagcttc<br>cgggatctgggatggcaggactggattatagcaccagaaggatacgctgcattttattgt<br>gatggagaatgttcttttccacttaacgcccatatgaatgccaccaaccacgctatagtt<br>cagactctggttcatctgatgtttcctgaccacgtaccaaagccttgttgtgctccaacc<br><br>aaattaaatgccatctctgttctgtactttgatgacagctccaatgtcattttgaaaaaa<br>tatagaaatatggtagtacgctcatgtggctgccactaa |

(continued)

| Seq. ID NO: | Nucleic Acid |
|---|---|
| 4(hB MP-6) | caacagagtcgtaatcgctctacccagtcccaggacgtggcgcgggtctccagtg cttca gattacaacagcagtgaattgaaaacagcctgcaggaagcatgagctgtatgtga gtttc caagacctgggatggcaggactggatcattgcacccaagggctatgctgccaatt actgt gatggagaatgctccttcccactcaacgcacacatgaatgcaaccaaccacgcga ttgtg cagaccttggttcaccttatgaaccccgagtatgtccccaaaccgtgctgtgcgc caact aagctaaatgccatctcggttctttactttgatgacaactccaatgtcattctga aaaaa tacaggaatatggttgtaagagcttgtggatgccactaa |
| 5(hB MP-7) | tccacggggagcaaacagcgcagccagaaccgctccaagacgcccaagaaccagg aagcc ctgcggatggccaacgtggcagagaacagcagcagcgaccagaggcaggcctgta agaag cacgagctgtatgtcagcttccgagacctgggctggcaggactggatcatcgcgc ctgaa ggctacgccgcctactactgtgaggggggagtgtgccttccctctgaactcctaca tgaac gccaccaaccacgccatcgtgcagacgctggtccacttcatcaacccggaaacgg tgccc aagccctgctgtgcgcccacgcagctcaatgccatctccgtcctctacttcgatg acagc tccaacgtcatcctgaagaaatacagaaacatggtggtccgggcctgtggctgcc actag |
| 6(hB MP-8) | gcagtgaggccactgaggaggaggcagccgaagaaaagcaacgagctgccgcagg ccaac cgactcccagggatctttgatgacgtccacggctcccacggccggcaggtctgcc gtcgg cacgagctctacgtcagcttccaggacctcggctggctggactgggtcatcgctc cccaa ggctactcggcctattactgtgaggggggagtgctccttcccactggactcctgca tgaat gccaccaaccacgccatcctgcagtccctggtgcacctgatgaagccaaacgcag tcccc aaggcgtgctgtgcacccaccaagctgagcgccacctctgtgctctactatgaca gcagc aacaacgtcatcctgcgcaagcaccgcaacatggtggtcaaggcctgcggctgcc actga |
| 7(hG DF-5) | gccccactggccactcgccagggcaagcgacccagcaagaaccttaaggctcgct gcagt |

(continued)

| Seq. ID NO: | Nucleic Acid |
|---|---|
|  | cggaaggcactgcatgtcaacttcaaggacatgggctgggacgactggatcatcg caccc cttgagtacgaggctttccactgcgaggggctgtgcgagttcccattgcgctccc acctg gagcccacgaatcatgcagtcatccagaccctgatgaactccatggaccccgagt ccaca ccacccacctgctgtgtgcccacgcggctgagtcccatcagcatcctcttcattg actct gccaacaacgtggtgtataagcagtatgaggacatggtcgtggagtcgtgtggct gcagg tag |
| 8(mG DF-6) | accgccttcgccagccgtcacggcaagcgacatggcaagaagtccaggctgcgct gcagc agaaagcctctgcacgtgaattttaaggagttaggctgggacgactggattatcg cgccc ctagagtacgaggcctatcactgcgagggcgtgtgcgactttccgctgcgctcgc acctt gagcccactaaccatgccatcattcagacgctgatgaactccatggacccgggct ccacc ccgcctagctgctgcgttcccaccaaactgactcccattagcatcctgtacatcg acgcg ggcaataatgtagtctacaagcagtatgaggacatggtggtggagtcctgcggct gtagg |
| 9(mG DF-7) | actgcgctggctgggactcggggagcgcagggaagcggtggtggcggcggtggcg gtggc ggcggcggcggcggcggcggcggcggcggcggcgcaggcaggggccacgggc gcaga ggccggagccgctgcagtcgcaagtcactgcacgtggactttaaggagctgggct gggac gactggatcatcgcgccattagactacgaggcataccactgcgagggcgtttgcg acttt cctctgcgctcgcacctggagcctaccaaccacgccatcattcagacgctgctca actcc atggcgcccgacgctgcgccagcctcctgctgcgtgcccgcaaggctcagtccca tcagc attctctacatcgatgccgccaacaacgtggtctacaagcagtacgaagacatgg tggtg gaggcctgcggctgcagg |

(continued)

| Seq. ID NO: | Nucleic Acid |
|---|---|
| 10 (hBM P-10) | aacgccaaaggaaactactgtaagaggaccccgctctacatcgacttcaaggagattggg<br><br>tgggactcctggatcatcgctccgcctggatacgaagcctatgaatgccgtggtgtttgt<br><br>aactacccctggcagagcatctcacacccacaaagcatgcaattatccaggccttggtc<br><br>cacctcaagaattcccagaaagcttccaaagcctgctgtgtgcccacaaagctagagccc<br><br>atctccatcctctatttagacaaaggcgtcgtcacctacaagtttaaatacgaaggcatg<br><br>gccgtctccgaatgtggctgtagatag |
| 11 (hGD F-2) | agcgccggggctggcagccactgtcaaaagacctccctgcgggtaaacttcgaggacatc<br><br>ggctgggacagctggatcattgcacccaaggagtatgaagcctacgagtgtaagggcggc<br><br>tgcttcttcccttggctgacgatgtgacgccgacgaaacacgctatcgtgcagaccctg<br><br>gtgcatctcaagttccccacaaaggtgggcaaggcctgctgtgtgcccaccaaactgagc<br><br>cccatctccgtcctctacaaggatgacatgggggtgcccaccctcaagtaccattacgag<br><br>ggcatgagcgtggcagagtgtgggtgcaggtag |

| Seq. ID NO: | Nucleic Acid |
|---|---|
| 12 (L51P ) | GCCGCCGCCGCCGTCGCCGCCGCCGGAGTCCTCGCCCCGCCGCGCTGCGCCCGGCTCGCGCTGCGC TAGT<br>CGCTCCGCTTCCCACACCCCGCCGGGGACTGGCAGCCGCCGCCGCACATCTGCCGCCACAGCCTCC GCCG<br>GCTACCCGAACGTTCTCGGGGCCAGCGCCGAGTGGATCACCGGGGACCGCGAGGCACCCGCGCGCC GCAG<br>ACCCCGCGCGGGCTGGAGCACCCGGCAGAGCGCGCCACAGCGCCGTGGCCTCTGCTGCCCGGGCTG CGCC<br>AGAGCCGCGGACGGGCGCGCAGAGCGCCGGGGACTCCGGAGCCGATCCCTAGCGCCGCGATGCGGA GCAC<br>CTACTGCAGGAGATCGGGGGCCTGGGACGCGCTGGCCGAGGTGTGATCGGACCCCAGGCTAGCCAC AAAG<br>GGCACTTGGCCCCAGGGCTAGGAGAGCGAGGGGAGAGCACAGCCACCCGCCTCGGCGGCCCGGGAC TCGG<br>CTCGACTCGCCGGAGAATGCGCCCGAGGACGACGGGGCGCCAGAGCCGCGGTGCTTTCAACTGGCG AGCG<br>CGAATGGGGGTGCACTGGAGTAAGGCAGAGTGATGCGGGGGGGCAACTCGCCTGGCACCGAGATCG CCGC<br>CGTGCCCTTCCCTGGACCCGGCGTCGCCCAGGATGGCTGCCCCGAGCCATGGGCCGCGGCGGAGCT AGCG<br>CGGAGCGCCCGACCCTCGACCCCCGAGTCCCGGAGCCGGCCCCGCGCGGGGCCACGCGTCCCTCGG GCGC<br>TGGTTCCTAAGGAGGACGACAGCACCAGCTTCTCCTTTCTCCCTTCCCTTCCCTGCCCCGCACTCC TCCC<br>CCTGCTCGCTGTTGTTGTGTGTCAGCACTTGGCTGGGGACTTCTTGAACTTGCAGGGAGAATAACT TGCG<br>CACCCCACTTTGCGCCGGTGCCTTTGCCCCAGCGGAGCCTGCTTCGCCATCTCCGAGCCCCACCGC CCCT<br>CCACTCCTCGGCCTTGCCCGACACTGAGACGCTGTTCCCAGCGTGAAAAGAGAGACTGCGCGGCCG GCAC<br>CCGGGAGAAGGAGGAGGCAAAGAAAAGGAACGGACATTCGGTCCTTGCGCCAGGTCCTTTGACCAG AGTT<br>TTTCCATGTGGACGCTCTTTCAATGGACGTGTCCCCGCGTGCTTCTTAGACGGACTGCGGTCTCCT AAAG<br>GTCGACCATGGTGGCCGGGACCCGCTGTCTTCTAGCGTTGCTGCTTCCCCAGGTCCTCCTGGGCGG CGCG<br>GCTGGCCTCGTTCCGGAGCTGGGCCGCAGGAAGTTCGCGGCGGCGTCGTCGGGCCGCCCCTCATCC CAGC<br>CCTCTGACGAGGTCCTGAGCGAGTTCGAGTTGCGGCTGCTCAGCATGTTCGGCCTGAAACAGAGAC CCAC<br>CCCCAGCAGGGACGCCGTGGTGCCCCCCTACATGCTAGACCTGTATCGCAGGCACTCAGGTCAGCC GGGC |

(continued)

| Seq. ID NO: | Nucleic Acid |
|---|---|
| | TCACCCGCCCCAGACCACCGGTTGGAGAGGGCAGCCAGCCGAGCCAACACTGTGCGCAGCTTCCACCATG<br>AAGAATCTTTGGAAGAACTACCAGAAACGAGTGGGAAAACAACCCGGAGATTCTTCTTTAATTTAAGTTC<br>TATCCCCACGGAGGAGTTTATCACCTCAGCAGAGCTTCAGGTTTTCCGAGAACAGATGCAAGATGCTTTA<br>GGAAACAATAGCAGTTTCCATCACCGAATTAATATTTATGAAATCATAAAACCTGCAACAGCCAACTCGA<br>AATTCCCCGTGACCAGACTTTTGGACACCAGGTTGGTGAATCAGAATGCAAGCAGGTGGGAAAGTTTTGA<br>TGTCACCCCCGCTGTGATGCGGTGGACTGCACAGGGACACGCCAACCATGGATTCGTGGTGGAAGTGGCC<br>CACTTGGAGGAGAAACAAGGTGTCTCCAAGAGACATGTTAGGATAAGCAGGTCTTTGCACCAAGATGAAC<br>ACAGCTGGTCACAGATAAGGCCATTGCTAGTAACTTTTGGCCATGATGGAAAAGGGCATCCTCTCCACAA<br>AAGAGAAAAACGTCAAGCCAAACACAAACAGCGGAAACGCCTTAAGTCCAGCTGTAAGAGACACCCTTTG<br>TACGTGGACTTCAGTGACGTGGGGTGGAATGACTGGATTGTGGCTCCCCCGGGGTATCACGCCTTTTACT<br>GCCACGGAGAATGCCCTTTTCCTCTGGCTGATCATCTGAACTCCACTAATCATGCCATTGTTCAGACGTT<br>GGTCAACTCTGTTAACTCTAAGATTCCTAAGGCATGCTGTGTCCCGACAGAACTCAGTGCTATCTCGATG<br>CTGTACCTTGACGAGAATGAAAAGGTTGTATTAAAGAACTATCAGGACATGGTTGTGGAGGGTTGTGGGT<br>GTCGCTAGTACAGCAAAATTAAATACATAAATATATATATATATATATATTTTAGAAAAAAGAAAAAAAC<br>AAACAAACAAAAAAACCCCACCCCAGTTGACACTTTAATATTTCCCAATGAAGACTTTATTTATGGAATG<br>GAATGGAAAAAAAAACAGCTATTTTGAAAATATATTTATATCTACGAAAAGAAGTTGGGAAAACAAATAT<br>TTTAATCAGAGAATTATTCCTTAAAGATTTAAAATGTATTTAGTTGTACATTTTATATGGGTTCAACCCC<br>AGCACATGAAGTATAATGGTCAGATTTATTTTGTATTTATTTACTATTATAACCACTTTTTAGGAAAAA<br>ATAGCTAATTTGTATTTATATGTAATCAAAAGAAGTATCGGGTTTGTACATAATTTTCCAAAAATTGTAG<br>TTGTTTTCAGTTGTGTGTATTTAAGATGAAAAGTCTACATGGAAGGTTACTCTGGCAAAGTGCTTAGCAC<br>GTTTGCTTTTTTGCAGTGCTACTGTTGAGTTCACAAGTTCAAGTCCAGAAAAAAAAAGTGGATAATCCAC<br>TCTGCTGACTTTCAAGATTATTATATTATTCAATTCTCAGGAATGTTGCAGAGTGATTGTCCAATCCATG<br>AGAATTTACATCCTTATTAGGTGGAATATTTGGATAAGAACCAGACATTGCTGATCTATTATAGAAACTC<br>TCCTCCTGCCCCTTAATTTACAGAAAGAATAAAGCAGGATCCATAGAAATAATTAGGAAAACGATGAACC<br>TGCAGGAAAGTGAATGATGGTTTGTTGTTCTTCTTTCCTAAATTAGTGATCCCTTCAAAGGGGCTGATCT<br>GGCCAAAGTATTCAATAAAACGTAAGATTTCTTCATTATTGATATTGTGGTCATATATATTTAAAATTGA<br>TATCTCGTGGCCCTCATCAAGGGTTGGAAATTTATTTGTGTTTTACCTTTACCTCATCTGAGAGCTCTTT<br>ATTCTCCAAAGAACCCAGTTTTCTAACTTTTTGCCCAACACGCAGCAAAATTATGCACATCGTGTTTTCT<br>GCCCACCCTCTGTTCTCTGACCTATCAGCTTGCTTTTCTTTCCAAGGTTGTGTGTTTGAACACATTTCTC<br>CAAATGTTAAACCTATTTCAGATAATAAATATCAAATCTCTGGCA |

(continued)

| Seq. ID NO: | Amino Acid |
|---|---|
| 13 (hBMP-2pre-protei n) | MVAGTRCLLALLLPQVLLGGAAGLVPELGRRKFAAASSGRPSSQPSDEVLSEFELRLLSMFG LKQRPTPS<br>RDAVVPPYMLDLYRRHSGQPGSPAPDHRLERAASRANTVRSFHHEESLEELPETSGKTTRRF FFNLSSIP<br>TEEFITSAELQVFREQMQDALGNNSSFHHRINIYEIIKPATANSKFPVTRLLDTRLVNQNAS RWESFDVT<br>PAVMRWTAQGHANHGFVVEVAHLEEKQGVSKRHVRISRSLHQDEHSWSQIRPLLVTFGHDGK GHPLHKRE<br>KRQAKHKQRKRLKSSCKRHPLYVDFSDVGWNDWIVAPPGYHAFYCHGECPFPLADHLSTNH AIVQTLVN<br>SVNSKIPKACCVPTELSAISMLYLDENEKVVLKNYQDMVVEGCGCR |
| 14 (hBMP-4) | SPKHHSQRARKKNKNCRRHSLYVDFSDVGWNDWIVAPPGYQAFYCHGDCPFP LADHLNST<br>NHAIVQTLVNSVNSSIPKACCVPTELSAISMLYLDEYDKVVLKNYQEMVVEGCGCR |
| 15 (hBMP-5) | NQNRNKSSSHQDSSRMSSVGDYNTSEQKQACKKHELYVSFRDLGWQDWIIAP EGYAAFYC<br>DGECSFPLNAHMNATNHAIVQTLVHLMFPDHVPKPCCAPTKLNAISVLYFDD SSNVILKK<br>YRNMVVRSCGCH |
| 16 (hBMP-6) | QQSRNRSTQSQDVARVSSASDYNSSELKTACRKHELYVSFQDLGWQDWIIAP KGYAANYC<br>DGECSFPLNAHMNATNHAIVQTLVHLMNPEYVPKPCCAPTKLNAISVLYFDD NSNVILKK<br>YRNMVVRACGCH |
| 17 (hBMP-7) | STGSKQRSQNRSKTPKNQEALRMANVAENSSSDQRQACKKHELYVSFRDLGW QDWIIAPE<br>GYAAYYCEGECAFPLNSYMNATNHAIVQTLVHFINPETVPKPCCAPTQLNAI SVLYFDDS<br>SNVILKKYRNMVVRACGCH |
| 18 (hBMP-8) | AVRPLRRRQPKKSNELPQANRLPGIFDDVHGSHGRQVCRRHELYVSFQDLGW LDWVIAPQ<br>GYSAYYCEGECSFPLDSCMNATNHAILQSLVHLMKPNAVPKACCAPTKLSAT SVLYYDSS<br>NNVILRKHRNMVVKACGCH |
| 19 (hGDF-5) | APLATRQGKRPSKNLKARCSRKALHVNFKDMGWDDWIIAPLEYEAFHCEGLC EFPLRSHL<br>EPTNHAVIQTLMNSMDPESTPPTCCVPTRLSPISILFIDSANNVVYKQYEDMVVESC<br>GCR |
| 20 (mGDF-6) | TAFASRHGKRHGKKSRLRCSRKPLHVNFKELGWDDWIIAPLEYEAYHCEGVC DFPLRSHL<br>EPTNHAIIQTLMNSMDPGSTPPSCCVPTKLTPISILYIDAGNNVVYKQYEDMVVESC<br>GCR |

(continued)

| Seq. ID NO: | Amino Acid |
|---|---|
| 21(mGDF -7) | TALAGTRGAQGSGGGGGGGGGGGGGGGGGGGGGAGRGHGRRGRSRCSRKSLHV DFKELGWD <br> DWIIAPLDYEAYHCEGVCDFPLRSHLEPTNHAIIQTLLNSMAPDAAPASCCV PARLSPIS <br> ILYIDAANNVVYKQYEDMVVEACGCR |
| 22 (hBMP-10) | NAKGNYCKRTPLYIDFKEIGWDSWIIAPPGYEAYECRGVCNYPLAEHLTPTK HAIIQALV <br> HLKNSQKASKACCVPTKLEPISILYLDKGVVTYKFKYEGMAVSECGCR |
| 23 (hGDF-2) | SAGAGSHCQKTSLRVNFEDIGWDSWIIAPKEYEAYECKGGCFFPLADDVTPT KHAIVQTL <br> VHLKFPTKVGKACCVPTKLSPISVLYKDDMGVPTLKYHYEGMSVAECGCR |
| 24 (L51P) | MVAGTRCLLALLLPQVLLGGAAGLVPELGRRKFAAASSGRPSSQPSDEV**P**SEFELRLLSMFG LKQRPTPS <br> RDAVVPPYMLDLYRRHSGQPGSPAPDHRLERAASRANTVRSFHHEESLEELPETSGKTTRRF FFNLSSIP <br> TEEFITSAELQVFREQMQDALGNNSSFHHRINIYEIIKPATANSKFPVTRLLDTRLVNQNAS RWESFDVT <br> PAVMRWTAQGHANHGFVVEVAHLEEKQGVSKRHVRISRSLHQDEHSWSQIRPLLVTFGHDGK GHPLHKRE <br> KRQAKHKQRKRLKSSCKRHPLYVDFSDVGWNDWIVAPPGYHAFYCHGECPFPLADHLNSTNH AIVQTLVN <br> SVNSKIPKACCVPTELSAISMLYLDENEKVVLKNYQDMVVEGCGCR |

**EXAMPLES**

Solvents, reagents and starting materials were purchased from commercial vendors and used as received unless otherwise described. All reactions were performed at room temperature unless otherwise stated. Starting materials were purchased from commercial sources or synthesised according to the methods described herein or using literature procedures.

**Abbreviations**

BMP2: Bone morphogenic protein 2

BMP2R: Bone morphogenic protein receptor

BV/CV: Bone volume relative to ceramics volume

βTCP: β tri-calcium phosphate

CAD : Computer-aided design

CV : Ceramics volume

Ex-Fix: External fixator

IVD: Intervertebral disc

K-wire: Kirschner wire

L51 P: BMP2 analogue - leucine replaced by proline at position 51

MSC: Mesenchymal stem cells

PBS: phosphate-buffered saline

PEEK: Polyether ether ketone

PEC: Polyelectrolyte complex

PMMA: Polymethyl methacrylate

ROI: Region of interest

s.c.: Subcutaneously

SOP: Standard operational protocol

VEGF: Vascular endothelial growth factor

$\mu$CT Micro computer tomography

**Materials**

Animals

The Wistar rats were either bought from Charles River Laboratories (Research Models and Services Germany GmbH, Sulzfeld, Germany) or were obtained from the centralised animal facility (CAF, Medical Faculty, University of Bern, Switzerland). The animals were all checked for health issues and were provided with an ISO-certified health report. Animals were kept for at least seven days prior the experiment to adaptation in the professional animal facility with *ad libidum* food and in standardised conditions.

**Analytical Methods**

2D digital X-rays

For all groups, true lateral and oblique anteroposterior X-rays of the surgical area were performed immediately post-operatively to confirm correct placement of the carrier and K-wires, as well as at six weeks and twelve weeks post-operatively to assess spinal fusion. These were collected using a digital X-ray system with 10s exposure time and 25kV (MX-20, Faxitron X-Ray Corporation, Edimex, Le Plessis, France). Postoperative and six weeks X-rays were taken in anaesthesia, and X-rays at twelve weeks were taken after sacrifice and hence did not need separate anaesthesia. The X-rays were taken at 25 kV and 10s exposure. For the X-ray at six weeks, rats were anaesthetised according to a balanced 1 anaesthetic protocol (Fentanyl 0.005 mg/kg and Medetomidine 0.15 mg/kg and Midazolam 2 mg/kg) mixed in the same syringe and administered s.c.. After s.c. injection of the anaesthetic mix, the rats were placed in a clean box and provided O$_2$ until loss of consciousness ensued (~ 5-10 min). After imaging the rats, the anaesthesia was reversed with a mix of Naloxone 0.12 mg/kg and Atipamezol 0.75 mg/kg and Flumazenil 0.2 mg/kg s.c. Animals recovered in clean cages until fully awake, and warmth was provided as needed. In all treatment groups, a tail sample of the surgical region (24pprox.. 5 cm long, including the fusion site and the adjacent vertebrae) was collected after sacrifice. For quantification the X-rays were scored by two independent experienced spine surgeons blinded to the study groups using the Bridwell criteria for spinal fusion outcome [32].

Micro Computer Tomography (wCT) Analysis

$\mu$CT was performed on 70% ethanol fixed samples after overnight fixation in 4% buffered formalin. The region of interest (ROI) of the samples was then scanned with a $\mu$-CT40 (SCANCO Medical, inc., Brüttisellen, Switzerland), using the built-in software from SCANCO Medical (SCANCO Module 64-bit; V5.15). The tails were oriented longitudinally to the axis of the X-ray beam. The X-ray tube was operated at 70 kVp and 57 mA, and the integration time was set at 300 ms with a voxel size of 10$\mu$m. The measurements were performed perpendicular to the longitudinal axis of the caudal (Cd) vertebrae, in a ROI between the two central screws of the fixation systems. To distinguish between soft and mineralised tissues, the tissue was segmented into two tissue types based on their greyscale (grey-level coded mineralisation density), that is < 200 Hounsfield unit (HU) for soft tissues and >200 HU for mineralised tissues ($\beta$TCP carrier, mineralised cartilaginous callus, woven and lamellar bone) [33]. For density and bone volume (BV) / total volume (TV) analyses, the TV was defined by manual segmentation (blinded to treatment groups), using the respective vertebral endplates of the fused segment as cranio-caudal, and the bone structure as circumferential limits. Analysis was carried out using the built-in software from SCANCO Medical (SCANCO Module 64-bit; V5.15). Carrier material resorption was quantified using 3D Slicer v5.0.3 (3D Slicer project, https://www.slicer.org), including the Segment Editor and Segment Statistics modules [34]. For Voxel-based analysis and differentiation, carrier material and bone were segmented manually for three different samples analysed in separate $\mu$CT scans, and average densities and standard deviations calculated. These showed congruent and consistent density distributions for all three samples and scans (means $\pm$ SD: bone density 7,938 $\pm$ 1,055 U; ceramic carrier 11,849 $\pm$ 931 U); allowing for application of a common threshold throughout all samples. Thresholds for density-based segmentation were set at 6,000 - 10,000 U for bone volume and 10,000 - 14,000 U for ceramic carrier volume, approximately equidistant and $\pm$ 2 SD ranges apart from the respective mean densities. The ROI for carrier resorption analysis were defined by manual segmentation, blinded to study groups and calibrated to a constant ratio relative to carrier size across scans [33]. Voxel counts for both segments (bone volume (BV) and carrier volume (CV)) were divided for each sample individually. Evaluation and semi-quantitative scoring of the X-ray and $\mu$CT images was done independently by experienced spine surgeons, blinded to the study groups. $\mu$CT images were viewed using the OsiriX image viewer (Pixmeo SARL, Bernex, Switzerland) and scored on an adapted version of the Bridwell criteria, as previously defined and detailed in the established standard operational protocol (SOP) by Oswald et al. [28].

Histology

After $\mu$CT scanning, tail samples were embedded in polymethylmethacrylate (PMMA, i.e., a mixture of methyl methacrylate, cat.# 55909 and Dibutyl Phthalate cat.# 524980, both from Sigma-Aldrich, inc., Buchs, Switzerland, and Perkadox 16 - cat. # G425.0100 Grogg Chemie, AG, Stettlen-Deisswil, Switzerland. Upon hardening (four-six weeks), the embedded tissue blocks were cut sagittally into approximately 600 $\mu$m thick ground sections using a slow-speed diamond saw (Varicut® VC50, Leco, St. Joseph, Mi, US) [35]. After mounting on acrylic glass slabs, the sections were ground and polished to a final thickness of -150 $\mu$m (KnuthRotor-3, Struers, S.A.S. Champigny sur Marne, Cedex, France) and surface stained with basic fuchsin and toluidine blue/McNeal [35,36]. All histology slides were subsequently digitalised by microphotography using a Keyence VHX-6000 microscope with an automated stage (Keyence International, inc., Urdorf, Switzerland). The digitalised slides were scored by independent raters, blinded to treatment groups, and rated regarding fusion success using a semi-quantitative score based on Emery *et al.* (1994) [37] and adapted to our animal model, see Table 2 and Figure 2.

*Table 2* - *The adapted histological fusion scores based on Emery et al. (1994) [37]*

| Score | Criteria |
| --- | --- |
| 0 | Carrier only, absence of bone-ingrowth |
| 1 | Partial bone-ingrowth, lacking bone bridge |
| 2 | Bone bridge present, <50% bridging bone |
| 3 | >50% bridging bone |
| 4 | Bone only |

Statistical Analysis

The number of animals per group was deemed to be sufficient based on power analysis. Assuming an effective size difference of 0.75 and a power of 0.80, N=5 per group was considered a suitable sample size. The data were analysed using GraphPad/Prism v10.0.0 for Mac OSX statistical analysis software, using the Kruskal-Wallis test for non-parametric data with Dunn's post-hoc analysis. A p-value <0.05 was considered statistically significant.

**Methods - Animal Experimental Procedures**

All animal experiments were carried out considering the ARRIVE guidelines and were carried out in accordance with the EU Directive 2010/63/EU for animal experiments. Permission for all described surgical procedures and applied drugs was obtained from the Swiss authorities of the canton of Bern (animal permit BE32/19). Elderly rats were used to best match the target patient population for treatment. In humans non-successful spinal fusion affects mostly the population over 70 years of age. Rats at the age of 12-18 months correspond to this age in humans and thus rats of this age group were used in this study.

The experimental procedure described here has been published as a SOP [28]. The current full study design is illustrated in Figure 1. It comprised a double-blinded randomised design and involved elderly Wistar rats (21 males, mean body weight 533.1 ± 148.8 g, mean ± SD, and 24 females, mean body weight 400.7 ± 52.7 g, respectively). The mean age of the rats was about 12 months to simulate the elderly population in humans. For brevity, the protocol is recapitalised below. Animals were randomly assigned to seven experimental groups, which consisted of phosphate-buffered saline (PBS) as material control, a "low-dose" (1 μg) and a "high-dose" of (10 μg) BMP2, a "high-dose" of the BMP2 analogue L51P (10 μg), 1 μg BMP2 and adding increasing doses of L51P, i.e., 1 μg, 5 μg, or 10 μg (see Figure 1 and Table 1).

*Table* 1 - *Experimental design and sample sizes of the in vivo elderly Wistar rat model. The last two columns show the lost animals due to complications per experimental group and sex.*

| Experimental group | | male | female | Sum | Lost animals male | Lost animals female |
|---|---|---|---|---|---|---|
| 1 | PBS | 4 | 4 | 8 | 2 | 0 |
| 2 | 1 μg BMP2 | 4 | 3 | 7 | 1 | 1 |
| 3 | 10 μg BMP2 | 3 | 3 | 6 | 1 | 0 |
| 4 | 1 μg BMP2 + 1 μg L51P | 2 | 5 | 7 | 4 | 0 |
| 5 | 1 μg BMP2 + 5 μg L51P | 3 | 3 | 6 | 1 | 0 |
| 6 | 1 μg BMP2 + 10 μg L51P | 3 | 3 | 6 | 0 | 0 |
| 7 | 10 μg L51P | 3 | 3 | 6 | 2 | 2 |
| | Total animals | 22 | 24 | 46 | 11 | 3 |

Preparation of cytokine-coated βTCP

The βTCP was produced at RMS foundation according to previously described protocols [22,25,26,29]. The cytokines BMP2 and L51P were obtained by Prof. Dr. emerit. Walter Sebald, University of Heidelberg, Germany. These were produced by over-expression in Escherichia coli and subsequent purification from extrusion bodies as described previously and were stored as lyophilised powder at -80°C prior usage [24,25,26]. Predefined amounts of BMP2 and L51P (Table 1) were dissolved in 25 μL of deionised water and adsorbed to beta-tricalcium phosphate (βTCP) carriers according to an established protocol under sterile conditions (see video of [28]). The βTCP was of the same quality and production (RMS foundation, Bettlach, Switzerland) as previously described with a porosity of about 75% [22]. The wetted ceramics were then air-dried overnight and implanted into the disc space after removal of the disc. The growth factor release of the βTCP carriers have already been studied and characterised in detail [29]. Eighty percent of the bound cytokine was "burst-released" after 4 days *in-vitro* in presence or absence of mouse calvaria osteoblasts [29].

Anaesthesia

Rats were anaesthetised with a balanced anaesthetic protocol (Fentanyl 0.005 mg/kg+ Medetomidine 0.15 mg/kg + Midazolam 2 mg/kg, all three from Sintetica S.A., Mendrisio, Switzerland, mixed in the same syringe and administered subcutaneously (s.c.). After s.c. injection of the anaesthetic mix, the rats were placed in a clean box and $O_2$ was provided until loss of consciousness (5-10 min). A surgical plane was granted for 45 min; 100% $O_2$ was provided via facemask throughout the surgery. If anaesthesia had to be prolonged after 45 min, it was continued with isoflurane (1.0-2.0% titrated to effect in 100% $O_2$) allowing for adequate depth of anaesthesia.

With a mechanistic and balanced approach, intra- and post-operative analgesia was provided by the epidural administration of 0.1 mL ropivacaine 0.5% between the vertebrae Cd1 and Cd2. Analgesia was calculated to last for about 3h. At the end of surgery, anaesthesia was reversed with a mix of Buprenorphine 0.05 mg/kg (Streuli Pharma SA, Uznach, Switzerland) + Atipamezol 0.75 mg/kg (Orion Corporation, Espoo, Finland) + Flumazenil 0.2 mg/kg s.c. (CPS Cito Pharma Services GmbH, Uster, Switzerland) (supplementary Video material in [28]). Meloxicam (1 mg/kg, Boehringer Ingelheim Basel, Switzerland) was administered s.c. at the end of surgery for continuous analgesia.

Surgery

The anesthetised animals were positioned in sternal recumbence. Epidural was clipped and disinfected with alcohol-based skin disinfection (chlorhexidine + 2% alcohol) at the surgical site. The epidural anaesthesia was performed between cauda (Cd)-2 and Cd-3 with aseptic technique (26G; 38mm spinal needle).

A posterior midline skin incision (length: 10 mm) over segment of interest was performed (Cd-4-5). This then followed a sharp dissection through the fascia exposing the two posterior longitudinal tendons. This step was important to protect the vessels during Kirschner (K)-wire insertion to avoid tail necrosis [30]. The surgery was then continued with blunt midline dissection between posterior longitudinal tendons to midline muscle. A sharp dissection of the muscle in the midline was performed. Then, an Ex-Fix made of polyether ether ketone (PEEK) rings was then mounted by insertion of K-wires. The identical design of the fixator was taken from the study by Martin *et al.* (2014) [31] using their computer-aided design (CAD) files. Insertion of two 0.8 K-wires per adjacent vertebra from left to the right with -wire drive through the Ex-Fix K-wire insertion cut-out. Lateral subfascial vessels were then visualised during K-wire insertion to avoid damage to the vessels and prevent postoperative tail necrosis [28,30,31]. The K-wires were then cut-off with sharp pliers at the level of the ring external fixator (Figure 1) [28]. For discectomy, the anulus fibrosis of the IVD was removed sharply from the endplates of the adjacent vertebrae. Nucleus pulposus material was then removed with a Kerrison rongeur and the endplate cartilage was debrided with a small, sharp curette (Figure 1). Then, cylindrical-shaped βTCP carriers with the dimensions 5 mm in ø and 2 mm in height were inserted into the disc niche. The filled-in "press-fit" βTCP served as "cage" and was compressed between the two vertebrae using the external ring fixator by fitting the threaded rods in a "press-fit" design. Skin incision was then closed subcutaneously using 4.0 vicryl and cutaneously with 5.0 Ethilone™ (Ethicon, Johnson & Johnson, ltd., Allschwil, Switzerland), and sterile dressing was then applied. The sutures were removed after ten days post-operatively, no anaesthesia was required.

Post-analgesia

Animals were allowed to recover until fully awake and warmth and $O_2$ was provided as needed. Buprenorphine analgesia (Streuli Pharma SA, Uznach, Switzerland) was continued in the first 24h post-op in drinking water (1mg/kg; 6 mL Temgesic® (Buprenorphine 0,3 mg/mL) + 360 mL drinking water and 10 mL of 5% Glucose) and with Meloxicam® 1 mg/kg (Sintetica S.A., Mendrisio, Switzerland) for four to seven days (one injection or oral application per day).

**EXAMPLE 1 - Spinal fusion by combined delivery of BMP2 and L51P**

X-ray data

The X-rays showed a clear trend that groups with a high dose of BMP2 had a very low spinal fusion Bridwell score showing strong spinal fusion (see, Figure 3, first row and Figure 4). Mixtures of low dose BMP2 with 1 μg and 5 μg L51P showed equally good spinal fusion scores as with high-dose BMP2 (Figure 4).

μCT analysis

The bone volume (BV) relative to ceramics volume (CV) (*i.e.*, BV/CV) revealed a clear up-regulation of the ratio in favour of more bone volume relative to the remaining CV when comparing material control (PBS) with addition of BMP2 in high doses (PBS vs 10 μg BMP2, P = 0.0074, see Figure 5), and if compared to all three mixtures of BMP2 with L51P, i.e. all with P ~ 0.01, see Figure 5). The quantification furthermore revealed the highest ratio towards bone if the mixture of 1 μg BMP2 and 1μg of L51P was used (Figures 3 and 5). Interestingly, L51P alone in higher doses did not have any significant effect compared to PBS control (Figure 3 middle row, and Fig. 5).
BV relative to total bolume (TV), i.e., BV/TV, analysis did not reveal significant differences between the study groups regarding bone volumes, total volumes of BV/TV ratios. Mineral density likewise was found to be similar across groups, with no statistically discernible differences at a mean across all specimens of 2.34 $\pm$ 0.13cm$^{-1}$.

Histology

Histological analysis confirmed the results derived from X-ray and μCT analysis The images showed a clear and robust ossification in samples with 10 μg BMP2, whereas for the PBS control almost no fusion was observed. Low-dose BMP2 exhibited an increase in ossification with partial bridging in some cases. Interestingly, mixtures of low-dose BMP2 and L51P revealed an equally strong ossification compared to high-dose BMP2, even for the lowest concentration of L51P added (Figure 4).

Inter-rater reliability, evaluated using Cohen's weighted kappa, yielded a kappa of 0.736 (SE = 0.027, z = 13.59, p < 0.001), indicating a strong agreement. Intra-rater reliability (assessed between two timepoints, three weeks apart) showed a kappa of 0.869 (SE = 0.046, z = 8.68, < 0.01), indicating a very strong agreement.

Ordinal logistic regression analysis did not show a significant influence of sex on the outcome of histology scores (coefficient = 1.18, SE = 0.81, t = 1.50) - suggesting a minimal to non-existent effect, especially considering the relative effect sizes of treatment groups (ranges: coefficients = 19.7 - 30.1, SE = 0.99 - 1.19, t = 16.2 - 24.7).

Discussion

The results demonstrate through X-ray, μCT and histology that applying a mixture of BMP2 and the BMP2-analogue L51) is sufficient to reduce the required dose of BMP2 without losing ossification benefits. A lower dose of BMP2 could help to reduce the frequent side effects encountered from high dose BMP application, particularly inspinal fusion surgeries.

**References**

1. R. Wittenauer, L. Smith, K. Aden, Background paper 6.12 osteoarthritis, World Health Organisation (WHO) (2013).
2. D.S. Chun, K.C. Baker, W.K. Hsu, Lumbar pseudarthrosis: a review of current diagnosis and treatment, Neurosurg Focus 39 (4) (2015) E10.
3. S.S. Rajaee, H.W. Bae, L.E. Kanim, R.B. Delamarter, Spinal fusion in the United States: analysis of trends from 1998 to 2008, Spine 37 (1) (2012) 67-76.
4. S.E. Broida, K. Murakami, A. Abedi, H.J. Meisel, P. Hsieh, J. Wang, A. Jain, Z. Buser, S.T. Yoon, AO Spine Knowledge Forum Degenerative, Clinical risk factors associated with the development of adjacent segment disease in patients undergoing ACDF: A systematic review, Spine J 23 (1) (2023) 146-156.
5. A. Matsugaki, M. Ito, Y. Kobayashi, T. Matsuzaka, R. Ozasa, T. Ishimoto, H. Takahashi, R. Watanabe, T. Inoue, K. Yokota, Y. Nakashima, T. Kaito, S. Okada, T. Hanawa, Y. Matsuyama, M. Matsumoto, H. Taneichi, T. Nakano, Innovative design of bone quality targeted intervertebral spacer: accelerated functional fusion guiding oriented collagen and apatite microstructure without autologous bone graft, Spine J 23 (4) (2023) 609-620.
6. E.N. Phan, W.K. Hsu, Novel Approaches Guiding the Future of Spinal Biologics for Bone Regeneration, Curr Rev Musculoskelet Med 15 (3) (2022) 205-212.
7. R. Watkins, R. Watkins, R. Hanna, Non-union rate with stand-alone lateral lumbar interbody fusion, Medicine (Baltimore) 93 (29) (2014) e275.
8. F. Veronesi, M. Sartori, C. Griffoni, M. Valacco, G. Tedesco, P.F. Davassi, A. Gasbarrini, M. Fini, G. Barbanti Brodano, Complications in Spinal Fusion Surgery: A Systematic Review of Clinically Used Cages, J Clin Med 11 (21) (2022).
9. B.I. Martin, S.K. Mirza, B.A. Comstock, D.T. Gray, W. Kreuter, R.A. Deyo, Reoperation rates following lumbar spine surgery and the influence of spinal fusion procedures, Spine 32 (3) (2007) 382-387.
10. N. Wakao, Y. Sakai, T. Watanabe, N. Osada, T. Sugiura, H. Iida, Y. Ozawa, K. Murotani, Spinal pseudoarthrosis following osteoporotic vertebral fracture: prevalence, risk factors, and influence on patients' activities of daily living 1 year after injury, Arch Osteoporos 18 (1) (2023) 45.
11. M.R. Urist, Bone: formation by autoinduction, Science 150 (3698) (1965) 893-899.
12. L. Fernandez, A. Petrizzo, The Use of Bone Morphogenetic Protein 2 (BMP-2) in Spine Surgery Is It Valuable? Bull Hosp Jt Dis (2013) 81 (1) (2023) 40-45.
13. M. D'Souza, N.A. Macdonald, J.L. Gendreau, P.J. Duddleston, A.Y. Feng, A.L. Ho, Graft Materials and Biologics for Spinal Interbody Fusion, Biomedicines 7 (4) (2019).
14. R.D. May, D.A. Frauchiger, C.E. Albers, A. Tekari, L.M. Benneker, F.M. Klenke, W. Hofstetter, B. Gantenbein, Application of Cytokines of the Bone Morphogenetic Protein (BMP) Family in Spinal Fusion - Effects on the Bone, Intervertebral Discs, and Mesenchymal Stromal Cells, Curr Stem Cell Res Ther 14 (8) (2019) 618-643.
15. A.M. Beschloss, C.M. DiCindio, J.S. Lombardi, J.N. Shillingford, J.L. Laratta, B. Holderread, P. Louie, A.J. Pugely, Z. Sardar, A.S. Khalsa, V.M. Arlet, C. Saifi, The Rise and Fall of Bone Morphogenetic Protein 2 Throughout the United States, Clin Spine Surg 35 (6) (2022) 264-269.
16. T. Makino, H. Tsukazaki, Y. Ukon, D. Tateiwa, H. Yoshikawa, T. Kaito, The Biological Enhancement of Spinal Fusion for Spinal Degenerative Disease, Int J Mol Sci 19 (8) (2018).
17. S.C. Chan, A. Tekari, L.M. Benneker, P.F. Heini, B. Gantenbein, Osteogenic differentiation of bone marrow stromal cells is hindered by the presence of intervertebral disc cells, Arthritis Res Ther 18 (1) (2015) 29.
18. A. Tekari, R.D. May, D.A. Frauchiger, S.C.W. Chan, L.M. Benneker, B. Gantenbein, The BMP2 variant L51P restores the osteogenic differentiation of human mesenchymal stromal cells in the presence of intervertebral disc cells , Eur Cell Mater 33 (2017) 197-210.
19. R.D. May, D.A. Frauchiger, C.E. Albers, L.M. Benneker, S. Kohl, B. Gantenbein, Inhibitory Effects of Human Primary Intervertebral Disc Cells on Human Primary Osteoblasts in a Co-Culture System, Int J Mol Sci 19 (4) (2018).
20. S.J. Brown, S.A. Turner, B.S. Balain, N.T. Davidson, S. Roberts, Is Osteogenic Differentiation of Human Nucleus Pulposus Cells a Possibility for Biological Spinal Fusion? Cartilage (2018) 1947603518754628.

21. D. Kok, C.M.M. Peeters, Z. Mardina, D.L.M. Oterdoom, S.K. Bulstra, A.G. Veldhuizen, R. Kuijer, F.H. Wapstra, Is remaining intervertebral disc tissue interfering with bone generation during fusion of two vertebrae? PLoS ONE 14 (4) (2019) e0215536.

22. M. Hauser, M. Siegrist, A. Denzer, N. Saulacic, J. Grosjean, M. Bohner, W. Hofstetter, Bisphosphonates reduce biomaterial turnover in healing of critical-size rat femoral defects, J Orthop Surg (Hong Kong) 26 (3) (2018) 2309499018802487.

23. S. Keller, J. Nickel, J.-L. Zhang, W. Sebald, T.D. Mueller, Molecular recognition of BMP-2 and BMP receptor IA, Nat Struct Mol Biol 11 (5) (2004) 481.

24. H.M. Khattab, M. Ono, W. Sonoyama, Y. Oida, S. Shinkawa, Y. Yoshioka, K. Maekawa, Y. Tabata, K. Sugama, W. Sebald, T. Kuboki, The BMP2 antagonist inhibitor L51P enhances the osteogenic potential of BMP2 by simultaneous and delayed synergism, Bone 69 (2014) 165-173.

25. H.J. Sebald, F.M. Klenke, M. S 1 iegrist, C.E. Albers, W. Sebald, W. Hofstetter, Inhibition of endogenous antagonists with an engineered BMP-2 variant increases BMP-2 efficacy in rat femoral defect healing, Acta Biomater 8 (10) (2012) 3816-3820.

26. C.E. Albers, W. Hofstetter, H.J. Sebald, W. Sebald, K.A. Siebenrock, F.M. Klenke, L51P - A BMP2 variant with osteoinductive activity via inhibition of Noggin, Bone 6 (3) (2012) 401-406.

27. H.M. Khattab, S. Kubota, M. Takigawa, T. Kuboki, W. Sebald, The BMP-2 mutant L51P: a BMP receptor IA binding-deficient inhibitor of noggin, J Bone Miner Metab 9 (2018).

28. K.A.C. Oswald, S.F. Bigdon, A.S. Croft, P. Bermudez-Lekerika, A. Bergadano, B. Gantenbein, C.E. Albers, Establishment of a Novel Method for Spinal Discectomy Surgery in Elderly Rats in an In Vivo Spinal Fusion Model, Meth & Prot 4 (4) (2021).

29. E. Wernike, W. Hofstetter, Y. Liu, G. Wu, H.J. Sebald, D. Wismeijer, E.B. Hunziker, K.A. Siebenrock, F.M. Klenke, Long-term cell-mediated protein release from calcium phosphate ceramics, J Biomed Mater Res A 92 (2) (2010) 463-474.

30. H. Gebhard, A.R. James, R.D. Bowles, J.P. Dyke, T. Saleh, S.P. Doty, L.J. Bonassar, R. Härtl, Biological intervertebral disc replacement: an in vivo model and comparison of two surgical techniques to approach the rat caudal disc, Evid Based Spine Care J 2 (1) (2011) 29-35.

31. J.T. Martin, A.H. Milby, J.A. Chiaro, D.H. Kim, N.M. Hebela, L.J. Smith, D.M. Elliott, R.L. Mauck, Translation of an engineered nanofibrous disc-like angle-ply structure for intervertebral disc replacement in a small animal model, Acta Biomater 10 (6) (2014) 2473-2481.

32. K.H. Bridwell, L.G. Lenke, K.W. McEnery, C. Baldus, K. Blanke, Anterior fresh frozen structural allografts in the thoracic and lumbar spine. Do they work if combined with posterior fusion and instrumentation in adult patients with kyphosis or anterior column defects? Spine 20 (12) (1995) 1410-1418.

33. M.L. Bouxsein, S.K. Boyd, B.A. Christiansen, R.E. Guldberg, K.J. Jepsen, R. Müller, Guidelines for assessment of bone microstructure in rodents using micro-computed tomography, J Bone Miner Res 25 (7) (2010) 1468-1486.

34. Kikinis RP, Vosburgh SD, Kirby K: 3D Slicer: A Platform for Subject-Specific Image Analysis, Visualization, and Clinical Support. In: Intraoperative Imaging and Image-Guided Therapy. Edited by Jolesz FA.Springer New York; 2014:277-289.

35. N.P. Lang, J.-C. Imber, K.N. Lang, B. Schmid, F. Muñoz, D.D. Bosshardt, N. Saulacic, Sequential osseointegration of a novel implant system based on 3D printing in comparison with conventional titanium implants, Clin Oral Implants Res (2023).

36. M.A. Abreu, L.G. Baroza, M.A. Rossi, Toluidine Blue-Basic Fuchsin Stain for Glycolmethacrylate Embedded Tissue, J Histotechnol (2013) 139-140.

37. S.E. Emery, M.S 1 . Brazinski, A. Koka, J.S. Bensusan, S. Stevenson, The biological and biomechanical effects of irradiation on anterior spinal bone grafts in a canine model, J Bone Joint Surg Am 76 (4) (1994) 540-548.

**Numbered Embodiments of the Invention**

1. A combination comprising an osteogenic bone morphogenic protein (BMP) and a BMP antagonist inhibitor for use in a method of spinal fusion in a subject, the method comprising locally applying the combination to the site of spinal fusion; wherein the BMP antagonist inhibitor is L51P.

2. The combination for use according to embodiment 1, wherein the osteogenic BMP is selected from BMP-2, BMP-4, BMP-5, BMP-7, and BMP-9.

3. The combination for use according to embodiment 1, wherein the osteogenic BMP is selected from BMP-2 and BMP-7.

4. The combination for use according to embodiment 1, wherein the osteogenic BMP is BMP-2.

5. The combination for use according to any one of embodiments 1 to 4, wherein the osteogenic BMP is a recombinant BMP, preferably a recombinant human BMP.

6. The combination for use according to any one of embodiments 1 to 5, wherein the BMP antagonist inhibitor is recombinant human L51P.

7. The combination for use according to embodiment 1, wherein the osteogenic BMP is recombinant human BMP-2 and the BMP antagonist inhibitor is recombinant human L51P.

8. The combination for use according to any one of embodiments 1 to 7, wherein the molar ratio of L51P to osteogenic BMP is less than 10:1.

9. The combination for use according to any one of embodiments 1 to 8, wherein the molar ratio of L51P to osteogenic BMP is less than or equal to about 5:1;
preferably wherein the molar ratio of L51P to osteogenic BMP is less than 3:1.

10. The combination for use according to any one of embodiments 1 to 9, wherein the osteogenic BMP is applied to the site of spinal fusion at a dose of less than 2 mg per spinal fusion,
for example a dose of from about 0.1 mg to about 0.5 mg per spinal fusion.

11. The combination for use according to any one of embodiments 1 to 10, wherein the osteogenic BMP and the BMP antagonist inhibitor are locally applied to the site of spinal fusion simultaneously or sequentially.

12. The combination for use according to any one of embodiments 1 to 11, wherein the osteogenic BMP and the BMP antagonist inhibitor are comprised in one or more pharmaceutical composition, for example wherein the pharmaceutical composition is selected from a liquid, gel, suspension, paste, putty, powder, granule, bead, an implant and a polyelectrolyte complex;
optionally wherein

(i) the osteogenic BMP and the BMP antagonist inhibitor are in separate pharmaceutical compositions; or

(ii) the osteogenic BMP and the BMP antagonist inhibitor are in a single pharmaceutical composition.

13. The combination for use according to embodiment 12, wherein the pharmaceutical composition is a controlled release pharmaceutical composition;

optionally wherein the pharmaceutical composition releases the osteogenic BMP and the BMP antagonist inhibitor over a period of at least 1-2 weeks.

14. The combination for use according to any one of embodiments 1 to 13, wherein the osteogenic BMP and the BMP antagonist inhibitor are applied to the intervertebral disc space between the vertebrae to be fused.

15. The combination for use according to any one of embodiments 1 to 14, wherein the method of spinal fusion further comprises inserting a spacer or carrier into the intervertebral disc space between the vertebrae to be fused.

16. The combination for use according to embodiment 15, wherein the spacer or carrier is selected from a solid implant, a cage, a porous matrix, a sponge, a scaffold, a bone allograft and a bone autograft.

17. The combination for use according to embodiment 15 or 16, wherein the spacer or carrier comprises an osteoconductive material.

18. The combination for use according to embodiment 17, wherein the osteoconductive material is selected from a calcium phosphate ceramic (e.g. hydroxyapaptite, tricalcium phosphate, or biphasic calcium phosphate), a porous calcium carbonate (e.g. a coral-derived ceramic), a bioactive glass, a collagen sponge, an osteoconductive metal (e.g. porous titanium), an osteoconductive polymer (e.g. poly-hydroxyethylmethacrylate), and an osteoconductive composite (e.g. a poly-epsilon caprolactone tricalcium phosphate composite or a composite comprising hydroxyapaptite granules and a hydrogel comprising a tricalcium phosphate microspheres).

19. The combination for use according to embodiment 17, wherein the spacer or carrier comprises β-tricalcium phosphate, for example wherein the β-tricalcium phosphate has a porosity of 70-90%.

20. The combination for use according to any one of embodiments 15 to 19, wherein the spacer or carrier further comprises the osteogenic BMP and the BMP antagonist inhibitor, preferably wherein the spacer or carrier is coated and/or infused with the osteogenic BMP and the BMP antagonist inhibitor.

21. The combination for use according to any one of embodiments 1 to 20, wherein the method of spinal fusion is selected from posterolateral interbody fusion, transforaminal interbody fusion, anterior interbody fusion and lateral lumbar interbody fusion;
preferably wherein the method of spinal fusion is anterior interbody fusion.

22. The combination for use of any one of embodiments 1 to 20, wherein the method of spinal fusion fuses two or more vertebrae selected from cervical vertebrae, thoracic vertebrae, lumber vertebrae and sacrum;
preferably, wherein the method of spinal fusion fuses two or more lumbar vertebrae or fuses the L5 and S1 vertebrae.

23. The combination for use according to any one of embodiments 1 to 22, wherein the method of spinal fusion further comprises laminectomy and/or discectomy.

24. The combination for use according to any one of embodiments 1 to 22, wherein the method of spinal fusion further comprises temporary immobilisation of the vertebrae to be fused;

optionally wherein the vertebrae to be fused are immobilised by one or more screws, rods, wires or plates;

preferably wherein the vertebrae to be fused are immobilised after application of the combination to the site of spinal fusion.

25. The combination for use according to any one of embodiments 1 to 24 wherein the composition prevents or inhibits one or more of pseudoarthrosis, soft-tissue swelling, radiculitis, pain at the site of spinal fusion or ectopic bone formation, osteolysis, potential carcinogenic effects, and dysphagia at the cervical level.

26. The combination for use according to any one of embodiments 1 to 25, wherein the subject is a human or a non-human mammal (e.g. a horse or a companion animal (e.g. a cat or dog)), preferably wherein the subject is a human.

27. The combination for use according to any one of embodiments 1 to 26, wherein the subject is a smoker and/or has a co-morbidity selected from a metabolic disease (e.g. diabetes, smoking, obesity, medication) or osteoporosis or malnutrition.

28. L51P for use in a method of spinal fusion in a subject, the method comprising locally applying the L51P to the site of spinal fusion concurrently (e.g. simultaneously or sequentially) with an osteogenic bone morphogenic protein (e.g. BMP-2).

29. An osteogenic bone morphogenic protein (e.g. BMP-2) for use in a method of spinal fusion in a subject, the method comprising locally applying the osteogenic bone morphogenic protein to the site of spinal fusion concurrently (e.g. simultaneously or sequentially) with L51P.

31. A combination product comprising an intervertebral spacer or carrier dimensioned for a human intervertebral disc-space, an osteogenic bone morphogenic protein (e.g. BMP-2), and a BMP antagonist inhibitor (e.g. L51P).

32. A kit comprising:

(i) an intervertebral spacer or carrier dimensioned for a human intervertebral disc-space;

(ii) an osteogenic bone morphogenic protein (e.g. BMP-2); and

(iii) a BMP antagonist inhibitor (e.g. L51P).

**Claims**

1. A combination comprising an osteogenic bone morphogenic protein (BMP) and a BMP antagonist inhibitor for use in a method of spinal fusion in a subject, the method comprising locally applying the combination to the site of spinal fusion; wherein the BMP antagonist inhibitor is L51P.

2. The combination for use according to claim 1, wherein the osteogenic BMP is selected from BMP-2, BMP-4, BMP-5, BMP-7, and BMP-9.

3. The combination for use according to any one of claims 1 to 2, wherein the BMP antagonist inhibitor is recombinant human L51P.

4. The combination for use according to claim 1, wherein the osteogenic BMP is recombinant human BMP-2 and the BMP antagonist inhibitor is recombinant human L51P.

5. The combination for use according to any one of claims 1 to 4, wherein the molar ratio of L51P to osteogenic BMP is less than 10:1, is less than or equal to about 5:1 or is less than 3:1.

6. The combination for use according to any one of claims 1 to 5, wherein the osteogenic BMP and the BMP antagonist inhibitor are comprised in one or more pharmaceutical composition, for example wherein the pharmaceutical composition is selected from a liquid, gel, suspension, paste, putty, powder, granule, bead, an implant and a polyelectrolyte complex; optionally wherein

   (i) the osteogenic BMP and the BMP antagonist inhibitor are in separate pharmaceutical compositions; or
   (ii) the osteogenic BMP and the BMP antagonist inhibitor are in a single pharmaceutical composition.

7. The combination for use according to any one of claims 1 to 6, wherein the method of spinal fusion further comprises inserting a spacer or carrier into the intervertebral disc space between the vertebrae to be fused.

8. The combination for use according to claim 7, wherein the spacer or carrier further comprises the osteogenic BMP and the BMP antagonist inhibitor, preferably wherein the spacer or carrier is coated and/or infused with the osteogenic BMP and the BMP antagonist inhibitor.
   preferably, wherein the method of spinal fusion fuses two or more lumbar vertebrae or fuses the L5 and S1 vertebrae.

9. The combination for use according to any one of claims 1 to 8 wherein the composition prevents or inhibits one or more of pseudoarthrosis, soft-tissue swelling, radiculitis, pain at the site of spinal fusion or ectopic bone formation, osteolysis, potential carcinogenic effects, and dysphagia at the cervical level.

10. The combination for use according to any one of claims 1 to 9, wherein the subject is a human or a non-human mammal (e.g. a horse or a companion animal (e.g. a cat or dog)), preferably wherein the subject is a human.

11. The combination for use according to any one of claims 1 to 10, wherein the subject is a smoker and/or has a co-morbidity selected from a metabolic disease (e.g. diabetes, smoking, obesity, medication) or osteoporosis or malnutrition.

12. L51P for use in a method of spinal fusion in a subject, the method comprising locally applying the L51P to the site of spinal fusion concurrently (e.g. simultaneously or sequentially) with an osteogenic bone morphogenic protein (e.g. BMP-2).

13. An osteogenic bone morphogenic protein (e.g. BMP-2) for use in a method of spinal fusion in a subject, the method comprising locally applying the osteogenic bone morphogenic protein to the site of spinal fusion concurrently (e.g. simultaneously or sequentially) with L51P.

**14.** A combination product comprising an intervertebral spacer or carrier dimensioned for a human intervertebral disc-space, an osteogenic bone morphogenic protein (e.g. BMP-2), and a BMP antagonist inhibitor (e.g. L51P).

**15.** A kit comprising:

(i) an intervertebral spacer or carrier dimensioned for a human intervertebral disc-space;
(ii) an osteogenic bone morphogenic protein (e.g. BMP-2); and
(iii) a BMP antagonist inhibitor (e.g. L51P).

Figure 1

**Example for Score 0:**
Carrier only, absence of bone
ingrowth

**Example for score 1:**
Partial bone ingrowth,
lacking bone bridge

**Example for score 2:**
Bone bridge present, <50%
bridging bone

**Example for score 3:**
>50% bridging bone

**Example for score 4:**
Bone only

## Figure 2

Figure 3A

Figure 3B

Figure 4

Figure 5

Figure 6

Europäisches
Patentamt

European
Patent Office

Office européen
des brevets

# EUROPEAN SEARCH REPORT

**Application Number**

EP 23 21 9776

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SEBALD H-J ET AL: "Inhibition of endogenous antagonists with an engineered BMP-2 variant increases BMP-2 efficacy in rat femoral defect healing", ACTA BIOMATERIALIA, ELSEVIER, AMSTERDAM, NL, vol. 8, no. 10, 1 October 2012 (2012-10-01), pages 3816-3820, XP002790675, ISSN: 1742-7061, DOI: 10.1016/J.ACTBIO.2012.06.036 | 1-6,9-13 | INV. A61K38/18 A61P19/08 |
| Y | * the whole document * | 7,8 | |
| A | KHATTAB HANY MOHAMED ET AL: "The BMP2 antagonist inhibitor L51P enhances the osteogenic potential of BMP2 by simultaneous and delayed synergism", BONE, PERGAMON PRESS., OXFORD, GB, vol. 69, 18 September 2014 (2014-09-18), pages 165-173, XP029091772, ISSN: 8756-3282, DOI: 10.1016/J.BONE.2014.09.011 * the whole document * | 1-13 | |
| A | "16th German Spine Congress Annual Meeting of the German Spine Society 9th to 11th December 2021 Muenster, Germany ED - Lamartina Claudio; Denaro Vincenzo; Faldini Cesare", EUROPEAN SPINE JOURNAL, SPRINGER BERLIN HEIDELBERG, BERLIN/HEIDELBERG, vol. 30, no. 11, 16 October 2021 (2021-10-16), pages 3328-3414, XP037598245, ISSN: 0940-6719, DOI: 10.1007/S00586-021-07017-6 [retrieved on 2021-10-16] * the whole document * | 1-13 | |

**TECHNICAL FIELDS SEARCHED (IPC)**

A61K
A61P

-/--

The present search report has been drawn up for all claims

| Place of search | Date of completion of the search | Examiner |
|---|---|---|
| The Hague | 7 May 2024 | Hars, Jesko |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 4

## EUROPEAN SEARCH REPORT

Application Number

EP 23 21 9776

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | RAHEL D. MAY: "Exogenous Stimulation of Human Intervertebral Disc Cells in 3-Dimensional Alginate Bead Culture With BMP2 and L51P: Cytocompatibility and Effects on Cell Phenotype", NEUROSPINE, vol. 17, no. 1, 31 March 2020 (2020-03-31), pages 77-87, XP093158343, ISSN: 2586-6583, DOI: 10.14245/ns.2040002.001 Retrieved from the Internet: URL:https://www.ncbi.nlm.nih.gov/pmc/artic les/PMC7136110/pdf/ns-2040002-001.pdf> * the whole document * | 1-13 | |
| A | CHRISTOPH E ALBERS ET AL: "L51P A BMP2 variant with osteoinductive activityinhibition of Noggin", BONE, PERGAMON PRESS., OXFORD, GB, vol. 51, no. 3, 21 June 2012 (2012-06-21), pages 401-406, XP028409221, ISSN: 8756-3282, DOI: 10.1016/J.BONE.2012.06.020 [retrieved on 2012-06-30] * the whole document * | 1-13 | |

TECHNICAL FIELDS
SEARCHED        (IPC)

-/--

The present search report has been drawn up for all claims

| Place of search | Date of completion of the search | Examiner |
|---|---|---|
| The Hague | 7 May 2024 | Hars, Jesko |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
...............................................................................
& : member of the same patent family, corresponding document

EPO FORM 1503 03.82 (P04C01)

page 2 of 4

Europäisches
Patentamt

European
Patent Office

Office européen
des brevets

# EUROPEAN SEARCH REPORT

**Application Number**

EP 23 21 9776

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | A TEKARI: "The BMP2 variant L51P restores the osteogenic differentiation of human mesenchymal stromal cells in the presence of intervertebral disc cells", EUROPEAN CELLS AND MATERIALS, vol. 33, 23 February 2017 (2017-02-23), pages 197-210, XP093158364, CH ISSN: 1473-2262, DOI: 10.22203/eCM.v033a15 Retrieved from the Internet: URL:https://www.ecmjournal.org/papers/vol033/pdf/v033a15.pdf> * the whole document * | 1-13 | |
| X | MICHEL HAUSER: "Bisphosphonates reduce biomaterial turnover in healing of critical-size rat femoral defects", JOURNAL OF ORTHOPAEDIC SURGERY, vol. 26, no. 3, 1 September 2018 (2018-09-01), XP093158353, ISSN: 2309-4990, DOI: 10.1177/2309499018802487 Retrieved from the Internet: URL:http://journals.sagepub.com/doi/full-xml/10.1177/2309499018802487> | 1-6,9-13 | |
| Y | * the whole document * | 7,8 | TECHNICAL FIELDS SEARCHED (IPC) |

-/--

The present search report has been drawn up for all claims

| Place of search | Date of completion of the search | Examiner |
|---|---|---|
| The Hague | 7 May 2024 | Hars, Jesko |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | KONRAD BACH: "Morphometric Analysis of Lumbar Intervertebral Disc Height: An Imaging Study", WORLD NEUROSURGERY, vol. 124, 1 April 2019 (2019-04-01), pages e106-e118, XP093158611, AMSTERDAM, NL ISSN: 1878-8750, DOI: 10.1016/j.wneu.2018.12.014 Retrieved from the Internet: URL:https://pdf.sciencedirectassets.com/280061/1-s2.0-S1878875019X00029/1-s2.0-S1878875018328365/main.pdf?X-Amz-Security-Token=IQoJb3JpZ2luX2VjEAcaCXVzLWVhc3QtMSJHMEUCIQDdjoY2ce6MhN2j6QKiwZxRamqFkNM1X4unyyDJOUn6xgIgazFXCKLvXMr4lvuO+k4es+yqY6WXxwKKBP1R3hzwUVxMqswUIYBAFGgwwNTkwMDM1NDY4NjUiDJBEr+PhArKTUZsmZ> * the whole document * | 7,8 | |
| | ----- | | |
| | | | TECHNICAL FIELDS SEARCHED (IPC) |

The present search report has been drawn up for all claims

| Place of search | Date of completion of the search | Examiner |
|---|---|---|
| The Hague | 7 May 2024 | Hars, Jesko |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**Application Number**

EP 23 21 9776

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

4(completely); 1-3, 5-13(partially)

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

The Search Division considers that the present European patent application does not comply with the requirements of unity of invention and relates to several inventions or groups of inventions, namely:

1. claims: 4(completely); 1-3, 5-13(partially)

   A combination comprising an osteogenic bone morphogenic protein (BMP) and a BMP antagonist inhibitor for use in a method of spinal fusion in a subject, the method comprising locally applying the combination to the site of spinal fusion; wherein the BMP antagonist inhibitor is L51P and wherein the BMP is BMP-2.
   L51P for use in a method of spinal fusion in a subject, the method comprising locally applying the L51P to the site of spinal fusion concurrently (e.g. simultaneously or sequentially) with an osteogenic bone morphogenic protein, wherein the osteogenic bone morphogenic protein is BMP-2.
   An osteogenic bone morphogenic protein for use in a method of spinal fusion in a subject, the method comprising locally applying the osteogenic bone morphogenic protein to the site of spinal fusion concurrently (e.g. simultaneously or sequentially) with L51P, wherein the osteogenic bone morphogenic protein is BMP-2.
   - - -

2-5. claims: 1-3, 5-15(all partially)

   A combination comprising an osteogenic bone morphogenic protein (BMP) and a BMP antagonist inhibitor for use in a method of spinal fusion in a subject, the method comprising locally applying the combination to the site of spinal fusion; wherein the BMP antagonist inhibitor is L51P.
   L51P for use in a method of spinal fusion in a subject, the method comprising locally applying the L51P to the site of spinal fusion concurrently (e.g. simultaneously or sequentially) with an osteogenic bone morphogenic protein.
   An osteogenic bone morphogenic protein for use in a method of spinal fusion in a subject, the method comprising locally applying the osteogenic bone morphogenic protein to the site of spinal fusion concurrently (e.g. simultaneously or sequentially) with L51P.
   A combination product comprising an intervertebral spacer or carrier dimensioned for a human intervertebral disc-space, an osteogenic bone morphogenic protein and a BMP antagonist inhibitor.
   A kit comprising:
   (i) an intervertebral spacer or carrier dimensioned for a human intervertebral disc-space;
   (ii) an osteogenic bone morphogenic protein; and
   (iii) a BMP antagonist inhibitor .
   For each of the embodiments, the BMP / osteogenic bone morphogenic protein is (invention number in brackets): BMP-4 (2), BMP-5 (3), BMP-7 (4), BMP-9 (5).
   - - -

page 1 of 2

Europäisches
Patentamt

European
Patent Office

Office européen
des brevets

**LACK OF UNITY OF INVENTION
SHEET B**

The Search Division considers that the present European patent application does not comply with the requirements of unity of invention and relates to several inventions or groups of inventions, namely:

6. claims: 14, 15(partially)

A combination product comprising an intervertebral spacer or carrier dimensioned for a human intervertebral disc-space, BMP-2, and a BMP antagonist inhibitor.
A kit comprising:
(i) an intervertebral spacer or carrier dimensioned for a human intervertebral disc-space;
(ii) BMP-2; and
(iii) a BMP antagonist inhibitor.
- - -

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2077759 A2

**Non-patent literature cited in the description**

- **WERNIKE et al.** VEGF incorporated into calcium phosphate ceramics promotes vascularisation and bone formation in vivo. *Eur Cell Mater*, February 2010, vol. 19, 30-40
- **R. WITTENAUER** ; **L. SMITH** ; **K. ADEN**. Background paper 6.12 osteoarthritis. *World Health Organisation (WHO)*, 2013
- **D.S. CHUN** ; **K.C. BAKER** ; **W.K. HSU**. Lumbar pseudarthrosis: a review of current diagnosis and treatment. *Neurosurg Focus*, 2015, vol. 39 (4), E10
- **S.S. RAJAEE** ; **H.W. BAE** ; **L.E. KANIM** ; **R.B. DELAMARTER**. Spinal fusion in the United States: analysis of trends from 1998 to 2008. *Spine*, 2012, vol. 37 (1), 67-76
- **S.E. BROIDA** ; **K. MURAKAMI** ; **A. ABEDI** ; **H.J. MEISEL** ; **P. HSIEH** ; **J. WANG** ; **A. JAIN** ; **Z. BUSER** ; **S.T. YOON**. AO Spine Knowledge Forum Degenerative, Clinical risk factors associated with the development of adjacent segment disease in patients undergoing ACDF: A systematic review. *Spine J*, 2023, vol. 23 (1), 146-156
- **A. MATSUGAKI** ; **M. ITO** ; **Y. KOBAYASHI** ; **T. MATSUZAKA** ; **R. OZASA** ; **T. ISHIMOTO** ; **H. TAKAHASHI** ; **R. WATANABE** ; **T. INOUE** ; **K. YOKOTA**. Innovative design of bone quality targeted intervertebral spacer: accelerated functional fusion guiding oriented collagen and apatite microstructure without autologous bone graft. *Spine J*, 2023, vol. 23 (4), 609-620
- **E.N. PHAN** ; **W.K. HSU**. Novel Approaches Guiding the Future of Spinal Biologics for Bone Regeneration. *Curr Rev Musculoskelet Med*, 2022, vol. 15 (3), 205-212
- **R. WATKINS** ; **R. WATKINS** ; **R. HANNA**. Non-union rate with stand-alone lateral lumbar interbody fusion. *Medicine (Baltimore)*, 2014, vol. 93 (29), e275
- **F. VERONESI** ; **M. SARTORI** ; **C. GRIFFONI** ; **M. VALACCO** ; **G. TEDESCO** ; **P.F. DAVASSI** ; **A. GASBARRINI** ; **M. FINI** ; **G. BARBANTI BRODANO**. Complications in Spinal Fusion Surgery: A Systematic Review of Clinically Used Cages. *J Clin Med*, 2022, vol. 11 (21)

- **B.I. MARTIN** ; **S.K. MIRZA** ; **B.A. COMSTOCK** ; **D.T. GRAY** ; **W. KREUTER** ; **R.A. DEYO**. Reoperation rates following lumbar spine surgery and the influence of spinal fusion procedures. *Spine*, 2007, vol. 32 (3), 382-387
- **N. WAKAO** ; **Y. SAKAI** ; **T. WATANABE** ; **N. OSADA** ; **T. SUGIURA** ; **H. LIDA** ; **Y. OZAWA** ; **K. MUROTANI**. Spinal pseudoarthrosis following osteoporotic vertebral fracture: prevalence, risk factors, and influence on patients' activities of daily living 1 year after injury. *Arch Osteoporos*, 2023, vol. 18 (1), 45
- **M.R. URIST**. Bone: formation by autoinduction. *Science*, 1965, vol. 150 (3698), 893-899
- **L. FERNANDEZ** ; **A. PETRIZZO**. The Use of Bone Morphogenetic Protein 2 (BMP-2) in Spine Surgery Is It Valuable?. *Bull Hosp Jt Dis*, 2013, vol. 81 (1), 40-45
- **M. D'SOUZA** ; **N.A. MACDONALD** ; **J.L. GENDREAU** ; **P.J. DUDDLESTON** ; **A.Y. FENG** ; **A.L. HO**. Graft Materials and Biologics for Spinal Interbody Fusion. *Biomedicines*, 2019, vol. 7 (4)
- **R.D. MAY** ; **D.A. FRAUCHIGER** ; **C.E. ALBERS** ; **A. TEKARI** ; **L.M. BENNEKER** ; **F.M. KLENKE** ; **W. HOFSTETTER** ; **B. GANTENBEIN**. Application of Cytokines of the Bone Morphogenetic Protein (BMP) Family in Spinal Fusion - Effects on the Bone, Intervertebral Discs, and Mesenchymal Stromal Cells. *Curr Stem Cell Res Ther*, 2019, vol. 14 (8), 618-643
- **A.M. BESCHLOSS** ; **C.M. DICINDIO** ; **J.S. LOMBARDI** ; **J.N. SHILLINGFORD** ; **J.L. LARATTA** ; **B. HOLDERREAD** ; **P. LOUIE** ; **A.J. PUGELY** ; **Z. SARDAR** ; **A.S. KHALSA**. The Rise and Fall of Bone Morphogenetic Protein 2 Throughout the United States. *Clin Spine Surg*, 2022, vol. 35 (6), 264-269
- **T. MAKINO** ; **H. TSUKAZAKI** ; **Y. UKON** ; **D. TATEIWA** ; **H. YOSHIKAWA** ; **T. KAITO**. The Biological Enhancement of Spinal Fusion for Spinal Degenerative Disease. *Int J Mol Sci*, 2018, vol. 19 (8)

- **S.C. CHAN** ; **A. TEKARI** ; **L.M. BENNEKER** ; **P.F. HEINI** ; **B. GANTENBEIN**. Osteogenic differentiation of bone marrow stromal cells is hindered by the presence of intervertebral disc cells. *Arthritis Res Ther*, 2015, vol. 18 (1), 29
- **A. TEKARI** ; **R.D. MAY** ; **D.A. FRAUCHIGER** ; **S.C.W. CHAN** ; **L.M. BENNEKER** ; **B. GANTENBEIN**. The BMP2 variant L51P restores the osteogenic differentiation of human mesenchymal stromal cells in the presence of intervertebral disc cells. *Eur Cell Mater*, 2017, vol. 33, 197-210
- **R.D. MAY** ; **D.A. FRAUCHIGER** ; **C.E. ALBERS** ; **L.M. BENNEKER** ; **S. KOHL** ; **B. GANTENBEIN**. Inhibitory Effects of Human Primary Intervertebral Disc Cells on Human Primary Osteoblasts in a Co-Culture System. *Int J Mol Sci*, 2018, vol. 19 (4)
- **S.J. BROWN** ; **S.A. TURNER** ; **B.S. BALAIN** ; **N.T. DAVIDSON** ; **S. ROBERTS**. Is Osteogenic Differentiation of Human Nucleus Pulposus Cells a Possibility for Biological Spinal Fusion?. *Cartilage*, 2018, 1947603518754628
- **D. KOK** ; **C.M.M. PEETERS** ; **Z. MARDINA** ; **D.L.M. OTERDOOM** ; **S.K. BULSTRA** ; **A.G. VELDHUIZEN** ; **R. KUIJER** ; **F.H. WAPSTRA**. Is remaining intervertebral disc tissue interfering with bone generation during fusion of two vertebrae?. *PLoS ONE*, 2019, vol. 14 (4), e0215536
- **M. HAUSER** ; **M. SIEGRIST** ; **A. DENZER** ; **N. SAULACIC** ; **J. GROSJEAN** ; **M. BOHNER** ; **W. HOFSTETTER**. Bisphosphonates reduce biomaterial turnover in healing of critical-size rat femoral defects. *J Orthop Surg (Hong Kong)*, 2018, vol. 26 (3), 2309499018802487
- **S. KELLER** ; **J. NICKEL** ; **J.-L. ZHANG** ; **W. SEBALD** ; **T.D. MUELLER**. Molecular recognition of BMP-2 and BMP receptor IA. *Nat Struct Mol Biol*, 2004, vol. 11 (5), 481
- **H.M. KHATTAB** ; **M. ONO** ; **W. SONOYAMA** ; **Y. OIDA** ; **S. SHINKAWA** ; **Y. YOSHIOKA** ; **K. MAEKAWA** ; **Y. TABATA** ; **K. SUGAMA** ; **W. SEBALD**. The BMP2 antagonist inhibitor L51P enhances the osteogenic potential of BMP2 by simultaneous and delayed synergism. *Bone*, 2014, vol. 69, 165-173
- **H.J. SEBALD** ; **F.M. KLENKE** ; **M. S 1 IEGRIST** ; **C.E. ALBERS** ; **W. SEBALD** ; **W. HOFSTETTER**. Inhibition of endogenous antagonists with an engineered BMP-2 variant increases BMP-2 efficacy in rat femoral defect healing. *Acta Biomater*, 2012, vol. 8 (10), 3816-3820
- **C.E. ALBERS** ; **W. HOFSTETTER** ; **H.J. SEBALD** ; **W. SEBALD** ; **K.A. SIEBENROCK** ; **F.M. KLENKE**. L51P - A BMP2 variant with osteoinductive activity via inhibition of Noggin. *Bone*, 2012, vol. 6 (3), 401-406
- **H.M. KHATTAB** ; **S. KUBOTA** ; **M. TAKIGAWA** ; **T. KUBOKI** ; **W. SEBALD**. The BMP-2 mutant L51P: a BMP receptor IA binding-deficient inhibitor of noggin. *J Bone Miner Metab*, 2018, vol. 9
- **K.A.C. OSWALD** ; **S.F. BIGDON** ; **A.S. CROFT** ; **P. BERMUDEZ-LEKERIKA** ; **A. BERGADANO** ; **B. GANTENBEIN** ; **C.E. ALBERS**. Establishment of a Novel Method for Spinal Discectomy Surgery in Elderly Rats in an In Vivo Spinal Fusion Model. *Meth & Prot*, 2021, vol. 4 (4)
- **E. WERNIKE** ; **W. HOFSTETTER** ; **Y. LIU** ; **G. WU** ; **H.J. SEBALD** ; **D. WISMEIJER** ; **E.B. HUNZIKER** ; **K.A. SIEBENROCK** ; **F.M. KLENKE**. Long-term cell-mediated protein release from calcium phosphate ceramics. *J Biomed Mater Res A*, 2010, vol. 92 (2), 463-474
- **H. GEBHARD** ; **A.R. JAMES** ; **R.D. BOWLES** ; **J.P. DYKE** ; **T. SALEH** ; **S.P. DOTY** ; **L.J. BONASSAR** ; **R. HÄRTL**. Biological intervertebral disc replacement: an in vivo model and comparison of two surgical techniques to approach the rat caudal disc. *Evid Based Spine Care J*, 2011, vol. 2 (1), 29-35
- **J.T. MARTIN** ; **A.H. MILBY** ; **J.A. CHIARO** ; **D.H. KIM** ; **N.M. HEBELA** ; **L.J. SMITH** ; **D.M. ELLIOTT** ; **R.L. MAUCK**. Translation of an engineered nanofibrous disc-like angle-ply structure for intervertebral disc replacement in a small animal model. *Acta Biomater*, 2014, vol. 10 (6), 2473-2481
- **K.H. BRIDWELL** ; **L.G. LENKE** ; **K.W. MCENERY** ; **C. BALDUS** ; **K. BLANKE**. Anterior fresh frozen structural allografts in the thoracic and lumbar spine. Do they work if combined with posterior fusion and instrumentation in adult patients with kyphosis or anterior column defects?. *Spine*, 1995, vol. 20 (12), 1410-1418
- **M.L. BOUXSEIN** ; **S.K. BOYD** ; **B.A. CHRISTIANSEN** ; **R.E. GULDBERG** ; **K.J. JEPSEN** ; **R. MÜLLER**. Guidelines for assessment of bone microstructure in rodents using micro-computed tomography. *J Bone Miner Res*, 2010, vol. 25 (7), 1468-1486
- 3D Slicer: A Platform for Subject-Specific Image Analysis, Visualization, and Clinical Support. **KIKINIS RP** ; **VOSBURGH SD** ; **KIRBY K**. Intraoperative Imaging and Image-Guided Therapy. 2014, 277-289
- **N.P. LANG** ; **J.-C. IMBER** ; **K.N. LANG** ; **B. SCHMID** ; **F. MUÑOZ** ; **D.D. BOSSHARDT** ; **N. SAULACIC**. Sequential osseointegration of a novel implant system based on 3D printing in comparison with conventional titanium implants. *Clin Oral Implants Res*, 2023
- **M.A. ABREU** ; **L.G. BAROZA** ; **M.A. ROSSI**. Toluidine Blue-Basic Fuchsin Stain for Glycolmethacrylate Embedded Tissue. *J Histotechnol*, 2013, 139-140

- **S.E. EMERY** ; **M.S 1 . BRAZINSKI** ; **A. KOKA** ; **J.S. BENSUSAN** ; **S. STEVENSON**. The biological and biomechanical effects of irradiation on anterior spinal bone grafts in a canine model. *J Bone Joint Surg Am*, 1994, vol. 76 (4), 540-548